# EUROPEAN PATENT APPLICATION

(11) **EP 4 269 603 A1**
(43) Date of publication of application: **01.11.2023**
(21) Application number: 22170965.2
(22) Date of filing: 29.04.2022
(51) Int. Cl.: C12N 15/86, C12N 15/87, C07K 14/16

(54) **TARGETED DNA INTEGRATION WITH LENTIVIRAL VECTORS AND USES THEREOF**

(71) Applicant: Universitat Pompeu Fabra, 08002 Barcelona (ES)
(72) Inventor: GÜELL CARGOL, Marc, 08002 Barcelona (ES); SÁNCHEZ-MEJÍAS GARCIA, Avencia, 08002 Barcelona (ES); IVANCIC, Dimitrije, 08002 Barcelona (ES)
(74) Representative: Icosa

(57) **Abstract**

The present invention relates to a composition of lentiviral particles comprising a mutated integrase and an RNA-guided nuclease, for targeted gene insertion.

## Description

### FIELD OF INVENTION

The invention relates to the field of gene editing and gene therapy, in particular targeted gene insertion.

### BACKGROUND OF INVENTION

Pathological genetic defects can range from a few bases to large deletions. Targeted DNA integration of gene-sized fragments in mammalian genomes can enable precise addition of therapeutic messages and new functionalities. Precise gene delivery methodologies based on non-homologous end joining (NHEJ) have been developed, such as homology-independent targeted integration (HITI). This methodology has been demonstrated for insertions of several kilobases, but remains inefficient for very large edits. While HITI might work to deliver exons, it may not be efficient enough to robustly deliver cDNAs of genes such as *DMD* (~14 kb) or *ABCA4* (~6.8 kb). HITI has been expanded to improve efficiency on DNA by fusion to DNA-binding domains recently. In bacteria, precise gene delivery has been demonstrated using CRISPR programmable transposons but this technology is not available for mammalian cells yet.

Development of novel methods for genome editing has enabled an expansion of human gene therapy (HGT) applications. Many HGT strategies are based on the addition of a DNA payload that can compensate for a genetic defect and/or provide the recipient cells with a synthetic feature. Delivery of therapeutic payloads has traditionally been based on both viral and non-viral vectors that uncontrollably integrate across the genome. Whilst precise delivery technologies are emerging, viral vector-mediated uncontrolled delivery has benefited an important number of patients. Insertional mutagenesis events have been observed, halting first attempts of gene therapy trials after cases of leukemia were linked to a retrovirus-based SCID-X1 gene therapy. Vector-driven clonal expansions have been observed again recently in CAR-T therapy, and in a leukemia case linked to the retroviral-based drug Strimvelis. rAAV can cause insertional mutagenesis and induce clonal expansions. Viral-free technologies such as the PiggyBac transposase (PB) have been linked to oncogenic induction. Lentiviral vectors are therapeutically used for uncontrolled insertion of transgenes both for *ex vivo* applications and *in vivo.* By mutating the integrase protein and incorporating SpCas9 nuclease in lentiviral particles we observe that lentiviral vectors can be reprogramed to precisely integrate transgenic DNA.

The discovery and implementation of precise delivery strategies using tools such as ZFN, TALENs and CRISPR-Cas9 can potentially avoid unintended gene disruption or activation and perform simultaneous gene inactivation and addition, which are key features for therapeutic genome modulation. Such techniques have also raised concerns related to safety. It has been reported that the integration of payloads can be imprecise, resulting in different unexpected integration outcomes, like integration of donors at off-target cut sites and genomic rearrangements such as inversions or translocations.

There is thus an unmet need for precise and safe gene delivery strategies, in particular for the delivery of very large sequences.

### SUMMARY

The present invention relates to a composition comprising:
(i) a nucleic acid encoding a lentiviral vector with impaired integration activity, optionally comprising an aptamer-binding protein,
(ii) a nucleic acid encoding a polypeptide or protein comprising an RNA-guided nuclease or nickase,
(iii) a nucleic acid encoding a guide RNA (gRNA) fused to an aptamer,
(iv) a nucleic acid encoding a transgene of interest, and
(v) optionally, a nucleic acid encoding a fusion protein comprising:
   - a first protein comprising or consisting of a GAG polyprotein,
   - a second protein comprising or consisting of an aptamer-binding protein, and
   - optionally, a third protein comprising or consisting of a POL polyprotein.

In some embodiments, the aptamer-binding protein is a MS2 bacteriophage coat protein (MCP), preferably sharing at least 75 % identity with SEQ ID NO: 61; and the aptamer is a MS2 RNA tetraloop binding sequence, preferably sharing at least 75 % identity with SEQ ID NO: 63.

In some embodiments, the RNA-guided nuclease or nickase is not fused to an integrase.

In some embodiments, the RNA-guided nuclease or nickase is a Cas protein.

In some embodiments, the RNA-guided nuclease or nickase is bound to a mutated hyperactive PiggyBac transposase.

In some embodiments, the integrase of (i) and/or the integrase comprised in the POL polyprotein of (v), when present, comprises at least one amino acid mutation at a position selected from the group consisting of 10, 11, 13, 64, 94, 116, 117, 119, 120, 122, 124, 128, 152, 164, 168, 170, 185, 186, 231, 264, 266 of the HIV-1 integrase of SEQ ID NO: 1 or at a corresponding position in another integrase.

In some embodiments, the integrase of (i) and/or of (v) comprises at least one amino acid mutation selected from the group consisting of D10K, E11K, E13K, D64A, D64E, G94D, G94E, G94R, G94K, D116A, D116E, N117D, N117E, N117R, N117K, S119A, S119P, S119T, S119G, S119D, S119E, S119R, S119K, N120D, N120E, N120R, N120K, T122K, T122I, T122V, T122A, T122R, A124D, A124E, A124R, A124K, A128T, E152A, E152D, D164N, Q168L, Q168A, E170G, F185K, K186E, R231G, R231K, R231D, R231E, R231S, K264R, K266R, and K273R of the HIV-1 integrase of SEQ ID NO: 1 or at a corresponding position in another integrase.

In some embodiments, the integration activity of the lentiviral vector of (i) is decreased or abolished by at least one mutation in the integrase sequence selected from the group consisting of a D116N substitution, a D164N substitution, an insertion of a premature stop codon, and a deletion of the integrase gene (ΔIN), numbering based on the HIV-1 integrase of SEQ ID NO: 1.

The present invention also relates to a method for producing lentiviral particles, comprising the steps of:
(i) transfecting lentiviral producer cells cultured in a suitable culture medium with the composition according to the invention, and
(ii) collecting lentiviral particles in the culture medium of said lentiviral producer cells.

The present invention also relates to a population of lentiviral particles obtainable by the method for producing lentiviral particles according to the invention.

The present invention also relates to a population of lentiviral particles comprising lentiviral particles comprising or consisting of:
a. lentiviral proteins and/or genes or parts thereof, wherein the lentiviral integrase has impaired integration activity,
b. an RNA-guided nuclease or nickase, preferably a Cas9 protein, or a nucleic acid sequence coding therefor,
c. a guide RNA fused to an aptamer, preferably to at least one MS2 RNA tetraloop-binding sequence, and
d. optionally, a fusion protein comprising (1) a first protein comprising or consisting of a GAG polyprotein, (2) a second protein comprising or consisting of an aptamer-binding protein, and optionally (3) a third protein comprising or consisting of a POL polyprotein, or a nucleic acid sequence coding therefor.

The present invention also relates to an *in vitro* method for site-specific integration of a transgene of interest into the genome of a cell, comprising infecting said cell with the population of lentiviral particles according to the invention, wherein the population of lentiviral particles comprises a transgene of interest or wherein a transgene of interest is delivered to the cell before, concomitantly with or after the population of lentiviral particles.

The present invention also relates to the population of lentiviral particles according to the invention, for use as a drug.

The present invention also relates to the population of lentiviral particles according to the invention, for use for site-specific integration of a transgene of interest into the genome of a cell, wherein the population of lentiviral particles comprises a transgene of interest or wherein a transgene of interest is to be delivered to the cell before, concomitantly with or after the population of lentiviral particles.

The present invention also relates to the population of lentiviral particles according to the invention, for use in the treatment of a disease in a subj ect in need thereof, preferably of a genetic disease, wherein the population of lentiviral particles comprises a transgene of interest or wherein a transgene of interest is to be delivered to the cell before, concomitantly with or after the population of lentiviral particles.

### DEFINITIONS

In this disclosure, any use of the singular forms **"a", "an"** or **"the"** includes the singular but also the plural references, unless the context clearly indicates otherwise. Thus, for example, a reference to "an agent" includes a single agent and a plurality of such agents.

**"Nucleic acid (sequence)"** and **"nucleotide sequence"** may be used interchangeably to refer to any molecule composed of, or comprising, monomeric nucleotides. A nucleic acid may be an oligonucleotide or a polynucleotide; it can be a DNA, an RNA, or a mix thereof. It can be chemically modified or artificial; *e.g.,* it encompasses peptide nucleic acids (PNA), morpholinos and locked nucleic acids (LNA), as well as glycol nucleic acids (GNA) and threose nucleic acid (TNA). Each of these nucleic acids distinguish from naturally occurring DNA or RNA by changes in the backbone of the molecule. Also, phosphorothioate nucleotides may be used. Other deoxynucleotide analogs include, without limitation, methylphosphonates, phosphoramidates, phosphorodithioates, N3'P5' phosphoramidates and oligoribonucleotide phosphorothioates and their 2'*O*-allyl analogs and 2'O-methylribonucleotide methylphosphonates which may be used in a nucleic acid of the disclosure.

**"Polypeptide", "peptide", "protein"** and **"amino acid sequence"** are used interchangeably to refer to a polymer of amino acid residues. Unless specified, a polymer of amino acid residues can be any length. The term also applies to amino acid polymers in which one or more amino acids are chemical analogues or modified derivatives of corresponding naturally-occurring amino acids.

"Nuclease" refers to an enzyme catalyzing the hydrolysis of nucleic acids within a nucleic acid sequence. Nuclease activity can result in single-stranded or double-stranded nucleic acid molecules break, wherein the nucleic molecule can be DNA or RNA.

**"Cas9"** or "**Cas9 nuclease"** refer to an RNA-guided nuclease comprising a Cas9 protein, or a fragment thereof *(e.g.,* a protein comprising an active or inactive DNA cleavage domain of Cas9, and/or the gRNA binding domain of Cas9). A Cas9 nuclease is also referred to sometimes as a casn1 nuclease or a CRISPR (clustered regularly interspaced short palindromic repeat)-associated nuclease. CRISPR is an adaptive immune system that provides protection against mobile genetic elements (viruses, transposable elements and conjugative plasmids). CRISPR clusters contain spacers, sequences complementary to antecedent mobile elements, and target invading nucleic acids. CRISPR clusters are transcribed and processed into CRISPR RNA (crRNA). In type II CRISPR systems, correct processing of pre-crRNA requires a trans-encoded small RNA (tracrRNA), endogenous ribonuclease 3 (rnc) and a Cas9 protein. The tracrRNA serves as a guide for ribonuclease 3-aided processing of pre-crRNA. Subsequently, the Cas9/crRNA/tracrRNA complex endonucleolytically cleaves linear or circular dsDNA target complementary to the spacer. The target strand not complementary to crRNA is first cut endonucleolytically, then trimmed 3'-5' exonucleolytically. In nature, DNA-binding and cleavage typically requires protein and both RNAs. However, single guide RNAs ("sgRNA" or simply "gRNA") can be engineered so as to incorporate aspects of both the crRNA and tracrRNA into a single RNA species.

Cas9 recognizes a short motif in the CRISPR repeat sequences (the PAM or protospacer adjacent motif) to help distinguish self vs. non-self. Cas9 nuclease sequences and structures are well known to those of skill in the art. Cas9 orthologs have been described in various species, including, but not limited to, *S. pyogenes* and *S. thermophilus.* Additional suitable Cas9 nucleases and sequences will be apparent to those of skill in the art based on this disclosure, and such Cas9 nucleases and sequences include Cas9 sequences from the organisms and loci disclosed in Chylinski et al., 2013. (RNA Biol. 10(5):726-37), the entire content of which is incorporated herein by reference.

In some embodiments, a Cas9 nuclease has an inactive (*e.g.,* an inactivated) DNA cleavage domain. A nuclease-inactivated Cas9 protein can interchangeably be referred to as a "dCas9" protein (for nuclease-"dead" Cas9). Methods for generating a Cas9 protein (or a fragment thereof) having an inactive DNA cleavage domain are known in the art (see, *e.g.,* Jinek et al., 2012. Science. 337(6096):816-821; Qi et al., 2013. Cell. 152(5): 1173-83, the entire content of each being incorporated herein by reference).

**"Exogenous"** refers to any molecule that is not naturally present in a cell or organism of interest, but which can be introduced thereinto by one or more genetic, biochemical or other methods. The natural presence of a molecule in a cell or organism may also be determined with respect to the particular developmental stage and environmental conditions thereof. Thus, for example, a molecule that is present only during embryonic development of muscle is an exogenous molecule with respect to an adult muscle cell. Similarly, a molecule induced by heat shock is an exogenous molecule with respect to a non-heat-shocked cell. An exogenous molecule can comprise, *e.g.,* a functioning version of a malfunctioning endogenous molecule or a malfunctioning version of a normally functioning endogenous molecule. By contrast, the term **"endogenous"** coins any molecule that is normally present in a cell or organism, at a particular developmental stage under particular environmental conditions.

**"Fusion protein"** refers to a single-chain hybrid polypeptide which comprises two or more amino acid sequences fused together (*i.e.,* from two or more different proteins and/or peptides). The two or more amino acid sequences can be fused together via a direct peptidic bond or indirectly through a peptidic linker. A fusion protein may be in particular fully encoded by a single nucleic acid sequence.

**"Gene"** typically refers to a DNA region encoding a protein (*i.e.,* a coding region). The term may also include DNA regions which do not *per se* encode a protein *(i.e.,* a non-coding region). The latter include, *e.g.,* regions transcribed into functional non-coding RNA molecules (*e.g.,* transfer RNA, ribosomal RNA, regulatory RNA, etc.). Other non-coding regions regulate the transcription and translation of coding regions (*i.e.,* regulatory elements), or serve as architectural elements (*e.g.,* scaffold/matrix attachment region), as origins of DNA replication, as centromeres or telomeres, etc. Regulatory elements include, without limitations, promoter sequences, terminators, translational regulatory sequences (*e.g.,* ribosome binding sites [RBS] and internal ribosome entry sites [IRES]), enhancers, silencers, insulators, boundary elements, replication origins, matrix attachment sites and locus control regions.

**"Eukaryotic"** refers to a cell or to an organism composed of such cells, which cells are not bacterial cells (prokaryotic cells). Eukaryotic cells include, without limitation, fungal cells, plant cells, and animal cells (*e.g.,* mammalian cells and human cells).

**"Transfection"** and any declension thereof refers to the introduction of one or several nucleic acid molecules (DNA and/or RNA) into one or more cells by non-viral means, whether *in vitro* or *in vivo.* Methods for transfection are well known in the art and include, *e.g*., lipofection and electroporation.

**"Transduction"** and any declension thereof refers to the introduction of one or more nucleic acid molecules (DNA and/or RNA) into one or more cells using a viral vector carrier, *e.g.,* a virus, a viral particle or a viral vector, including without limitation, retroviruses (including lentiviruses), adenoviruses, adeno-associated viruses (AAV), and vectors derived thereof.

**"Cleavage"** refers to the breakage of the covalent backbone of a DNA molecule. Cleavage can be initiated by a variety of methods including, but not limited to, enzymatic or chemical hydrolysis of a phosphodiester bond. Both single-stranded cleavage and double-stranded cleavage are possible, and double-stranded cleavage can occur as a result of two distinct single-stranded cleavage events. DNA cleavage can result in the production of either blunt ends or staggered ends.

**"Specificity"** refers to the ability to selectively bind a sequence which shares a degree of sequence identity to a selected sequence.

**"Insertion"** and **"integration"** refer to the addition of a nucleic acid sequence into a second nucleic acid sequence or into a genome or a portion thereof. Insertion may be "specific", "site-specific", "targeted" or "on-targeted": these adjectives define the insertion of a nucleic acid into a specific site of a second nucleic acid or into a specific site of a genome or a portion thereof *(i.e.,* a site that has been purposely selected for insertion). Conversely, the adjectives "random", "non-targeted" or "off-targeted" refer to non-specific and/or unintended insertion of a nucleic acid into an unwanted site. The terms "total" or "overall" refer to the total number of insertions.

**"Mutation"** refers to a substitution of a residue within a sequence, e.g., a nucleic acid or amino acid sequence, with another residue; and/or to a deletion or insertion of one or more residues within a nucleic acid or amino acid sequence. Mutations are typically described herein by identifying the original residue followed by the position of the residue within the sequence, then the identity of the newly substituted residue. Various methods for making amino acid substitutions (mutations) provided herein are well known in the art, and are provided by, for example, Green & Sambrook, 2012 (Molecular cloning: α laboratory manual (4th Ed.). Cold Spring Harbor Laboratory Press, Cold Spring Harbor, N.Y.).

**"Integrase"** refers to an enzyme produced by the genome of retroviruses (*e.g.,* HIV) that catalyzes the integration of a nucleic acid sequence within the genome of a host cell. As opposed to transiently-expressed nucleic acid sequences, such as transfected plasmids, integrated nucleic acid sequences are stably expressed by the cell regardless of mitosis events, and are replicated along with the rest of the genome.

**"Transposase"** refers to an enzyme that binds to the end of a transposon and catalyzes its movement to another part of the genome by a cut-and-paste mechanism, or a replicative transposition mechanism.

**"Mutated",** in connection with a sequence (*e.g.,* an amino acid sequence or a nucleic acid sequence) means that the sequence is different than a reference sequence, such as a wild-type sequence. Typically, a mutated sequence comprises at least one of a substitution, an addition or a deletion of one or several residues by comparison to a reference sequence, such as a corresponding wild-type sequence.

**"Linker"** refers to a chemical group or a molecule linking two adjacent molecules or moieties.

**"Identity"** or **"identical",** when used in a relationship between the sequences of two or more amino acid sequences, or of two or more nucleic acid sequences, refers to the degree of sequence relatedness between amino acid sequences or nucleic acid sequences, as determined by the number of matches between strings of two or more amino acid residues or nucleic acid residues. "Identity" measures the percent of identical matches between the smaller of two or more sequences with gap alignments (if any) addressed by a particular mathematical model or computer program (*i.e.,* "algorithms"). Identity of related amino acid sequences or nucleic acid sequences can be readily calculated by known methods. Such methods include, but are not limited to, those described in Lesk A. M. (1988). Computational molecular biology: Sources and methods for sequence analysis. New York, NY: Oxford University Press; Smith D. W. (1993). Biocomputing: Informatics and genome projects. San Diego, CA: Academic Press; Griffin A. M. & Griffin H. G. (1994). Computer analysis of sequence data, Part 1. Totowa, NJ: Humana Press; von Heijne G. (1987). Sequence analysis in molecular biology: treasure trove or trivial pursuit. San Diego, CA: Academic press; Gribskov M. R. & Devereux J. (1991). Sequence analysis primer. New York, NY: Stockton Press; Carillo et al., 1988. SIAM J Appl Math. 48(5): 1073-82. Preferred methods for determining identity are designed to give the largest match between the sequences tested. Methods of determining identity are described in publicly available computer programs. Preferred computer program methods for determining identity between two sequences include the GCG program package, including GAP (Genetics Computer Group, University of Wisconsin, Madison, WI; Devereux et al., 1984. Nucleic Acids Res. 12(1 Pt 1):387-95), BLASTP, BLASTN, and FASTA (Altschul et al., 1990. J Mol Biol. 215(3):403-10). The BLASTX program is publicly available from the National Center for Biotechnology Information (NCBI) and other sources (BLAST Manual, Altschul et al. NCB/NLM/NIH Bethesda, Md. 20894). The well-known Smith Waterman algorithm may also be used to determine identity.

**"Subject"** refers to a mammal, preferably a human. A subject may be a **"patient",** *i.e.,* a warm-blooded animal, more preferably a human, who/which is awaiting the receipt of, or is receiving medical care or was/is/will be the object of a medical procedure, or is monitored for the development of a disease. The term **"mammal"** refers here to any mammal, including humans, domestic and farm animals, and zoo, sports, or pet animals, such as dogs, cats, cattle, horses, sheep, pigs, goats, rabbits, etc. Preferably, the mammal is a primate, more preferably a human.

**"Treatment", "alleviation", "curation"** and any declensions thereof refer to a therapeutic treatment, excluding prophylactic or preventative measures; wherein the object is to slow down, lessen, stop or even reverse (either partially or totally) the evolution of a targeted pathologic condition or disorder. Those in need of treatment include those already with the disorder as well those suspected to have the disorder. A subject is successfully "treated" for the targeted pathologic condition or disorder if, after receiving treatment, they show observable and/or measurable reduction in or absence of one or more symptoms associated with the pathologic condition or disorder; relief to some extent; reduced morbidity and/or mortality; and/or improvement in quality-of-life issues. The above parameters for assessing successful treatment and improvement in the disease are readily measurable by routine procedures familiar to a physician.

**"Prevention"** and any declension thereof refers to prophylactic and preventative measures, wherein the object is to reduce the chances that a subject will develop a given pathologic condition or disorder over a given period of time. Such a reduction may be reflected, *e.g.,* in a delayed onset of at least one symptom of the pathologic condition or disorder in the subject.

### DETAILED DESCRIPTION

The present invention relates to a composition comprising:
(i) a nucleic acid encoding a lentiviral vector with impaired integration activity, optionally comprising an aptamer-binding protein,
(ii) a nucleic acid encoding a polypeptide or protein comprising an RNA-guided nuclease or nickase,
(iii) a nucleic acid encoding a guide RNA (gRNA) fused to an aptamer,
(iv) a nucleic acid encoding a transgene of interest, and
(v) optionally, a nucleic acid encoding a fusion protein comprising:
   - a first protein comprising or consisting of a GAG polyprotein,
   - a second protein comprising or consisting of an aptamer-binding protein; and
   - optionally, a third protein comprising or consisting of a POL polyprotein.

In some embodiments, the nucleic acids of the composition are deoxyribonucleic acids (DNA), ribonucleic acids (RNA), or combinations thereof. In some embodiments, the nucleic acids of the composition are DNA. In some embodiments, the nucleic acids of the composition are RNA.

In some embodiments, the nucleic acids of the composition comprise or consist of natural nucleotides, non-natural nucleotides, or combinations thereof. In some embodiments, the nucleic acids of the composition comprise or consist of natural nucleotides. In some embodiments, the natural nucleotides are selected from the group comprising or consisting of adenine, guanine, cytosine, thymine and uracil. In some embodiments, the natural nucleotides are selected from the group comprising or consisting of adenine, guanine, cytosine and thymine. In some embodiments, the natural nucleotides are selected from the group comprising or consisting of adenine, guanine, cytosine and uracil. In some embodiments, the nucleic acids of the composition comprise or consist of non-natural nucleotides (*e.g.,* chemically modified).

In some embodiments, the nucleic acids of the composition are single-stranded, double-stranded, or combinations thereof. In some embodiments, the nucleic acids of the composition are single-stranded. In some embodiments, the nucleic acids of the composition are double-stranded.

In some embodiments, the nucleic acids of the composition are selected from the group comprising or consisting of a plasmid, a messenger RNA (mRNA), a naked DNA, a cosmid, a fosmid, a prokaryotic chromosome (*e.g.,* bacterial artificial chromosome), a eukaryotic chromosome (*e.g.,* yeast artificial chromosome or human artificial chromosome), or combinations thereof. In some embodiments, the nucleic acids of the composition are plasmids, mRNAs or combinations thereof. In some embodiments, the nucleic acids of the composition are plasmids. In some embodiments, the nucleic acids of the composition are mRNAs.

In some embodiments, each nucleic acid of the composition is comprised in its own vector (such as, *e.g.,* a plasmid, a cosmid, a fosmid, a chromosome, etc.). Alternatively, two nucleic acids of the composition may be comprised in a single vector. For instance, the nucleic acids of (i) and (ii), or (i) and (iii), or (i) and (iv), or (i) and (v), or (ii) and (iii), or (ii) and (iv), or (ii) and (v), or (iii) and (iv), or (iii) and (v), or (iv) and (v) may be comprised in a single vector. Alternatively, three nucleic acids of the composition may be comprised in a single vector. For instance, the nucleic acids of (i) and (ii) and (iii), or (i) and (ii) and (iv), or (i) and (ii) and (v), or (i) and (iii) and (iv), or (i) and (iii) and (v), or (i) and (iv) and (v), or (ii) and (iii) and (iv), or (ii) and (iii) and (v), or (ii) and (iv) and (v), or (iii) and (iv) and (v) may be comprised in a single vector. Alternatively, four nucleic acids of the composition may be comprised in a single vector. For instance, the nucleic acids of (i) and (ii) and (iii) and (iv), or (i) and (ii) and (iii) and (v), or (i) and (ii) and (iv) and (v), or (i) and (iii) and (iv) and (v), or (ii) and (iii) and (iv) and (v) may be comprised in a single vector. Alternatively, the five nucleic acids of the composition may be comprised in a single vector.

In some embodiments, the nucleic acids of the composition comprise at least one coding sequence, and optionally at least one non-coding sequence, *e.g.,* a regulatory sequence or a replication origin sequence.

In some embodiments, the nucleic acids of the composition encode proteins (including polyproteins and pro-polypeptides that are post-translationally cleaved and/or modified), nucleic acids, or a combination thereof. In other words, the nucleic acids of the composition comprise at least one expression cassette.

The proteins or nucleic acids encoded by the nucleic acids of the composition are further described herein.

According to the invention, the composition comprises a lentiviral vector with impaired integration activity.

As used herein, the expression **"impaired integration activity"** means that the integrase of the lentiviral vector has a decreased or abolished integration activity by comparison to a wild-type lentiviral vector. Preferably, the lentiviral vector has an abolished integration activity by comparison to a wild-type lentiviral vector. In order to achieve a decreased or abolished integration activity, the integrase of the lentiviral vector is genetically modified, *e.g.*, with one or several amino acid substitutions altering its integration activity, or by partial or complete deletion of the integrase-coding sequence.

In some embodiments, the integrase of the lentiviral vector comprises at least one amino acid mutation compared to a wild-type (WT) integrase (*e.g.,* as compared to the wild-type HIV-1 integrase with SEQ ID NO: 1). As used herein, the term "at least one" means 1, 2, 3, 4, 5, 6, 7, 8, 9, 10 or more. As used herein, the term "amino acid mutation" encompasses substitutions, additions, deletions and the like.

In the following, amino acid residue numbering is based on the sequence of the HIV-1 integrase with SEQ ID NO: 1, unless stated otherwise. The skilled artisan can readily determine corresponding positions in the sequence of other integrases, *e.g*., by sequence alignment.

| **SEQ ID NO: 1 - HIV-1 integrase** |
|---|
| FLDGIDKAQEEHEKYHSNWRAMASDFNLPPVVAKEIVASCDKCQLKGEAMHG QVDCSPGIWQLDCTHLEGKVILVAVHVASGYIEAEVIPAETGQETAYFLLKLAG RWPVKTVHTDNGSNFTSTTVKAACWWAGIKQEFGIPYNPQSQGVIESMNKELK KIIGQVRDQAEHLKTAVQMAVFIHNFKRKGGIGGYSAGERIVDIIATDIQTKELQ KQITKIQNFRVYYRDSRDPVWKGPAKLLWKGEGAVVIQDNSDIKVVPRRKAKII RDYGKQMAGDDCVASRQDED |

In some embodiments, the integrase of the lentiviral vector comprises at least one amino acid mutation at positions selected from the group comprising or consisting of 10, 11, 13, 64, 94, 116, 117, 119, 120, 122, 124, 128, 152, 164, 168, 170, 185, 186, 231, 264, 266, and 273.

In some embodiments, the integrase of the lentiviral vector comprises at least one amino acid mutation selected from the group comprising or consisting of E10K, E11K, E13K, D64V, D64A, D64E, G94D, G94E, G94R, G94K, D116N, D116A, D116E, N117D, N117E, N117R, N117K, S119A, S119P, S119T, S119G, S119D, S119E, S119R, S119K, N120D, N120E, N120R, N120K, T122K, T122I, T122V, T122A, T122R, A124D, A124E, A124R, A124K, A128T, E152V, E152A, E152D, D164N, Q168L, Q168A, E170G, F185K, K186Q, K186E, R231G, R231K, R231D, R231E, R231S, K264R, K266R, and K273R.

In some embodiments, the integrase of the lentiviral vector comprises at least one amino acid mutation at positions selected from the group comprising or consisting of 10, 13, 64, 116, 119, 152, 164, 186 and 231. In some embodiments, the integrase of the lentiviral vector comprises at least one amino acid mutation selected from the group comprising or consisting of E10K, E13K, D64V, D64A, D64E, D116N, D116A, D116E, S119A, S119P, S119T, S119G, S119D, S119E, S119R, S119K, E152V, E152A, E152D, D164N, K186Q, K186E, R231G, R231K, R231D, R231E and R231S.

In some embodiments, the integrase of the lentiviral vector comprises at least one amino acid mutation selected from the group comprising or consisting of E10K, E13K, D64V, D116N, S119G, E152V, D164N, K186Q and R231G.

In some embodiments, the integrase of the lentiviral vector comprises at least one mutation that disrupt interaction with p75. In some embodiments, the integrase of the lentiviral vector comprises at least one amino acid mutation selected from the group comprising or consisting of the double mutation E10K/E13K, and K186Q. In some embodiments, the integrase of the lentiviral vector comprises the amino acid mutations E10K and E13K, typically the integrase of the lentiviral vector has the amino acid sequence of SEQ ID NO: 2. In some embodiments, the integrase of the lentiviral vector comprises the amino acid mutation K186Q, typically the integrase of the lentiviral vector has the amino acid sequence of SEQ ID NO: 8.

In some embodiments, the integrase of the lentiviral vector comprises at least one mutation in its catalytic site. In some embodiments, the integrase of the lentiviral vector comprises at least one amino acid mutation selected from the group comprising or consisting of D64V, D116N and E152V. In some embodiments, the integrase of the lentiviral vector comprises the amino acid mutation D64V, typically the integrase of the lentiviral vector has the amino acid sequence of SEQ ID NO: 3. In some embodiments, the integrase of the lentiviral vector comprises the amino acid mutation D116N, typically the integrase of the lentiviral vector has the amino acid sequence of SEQ ID NO: 4. In some embodiments, the integrase of the lentiviral vector comprises the amino acid mutation E152V, typically the integrase of the lentiviral vector has the amino acid sequence of SEQ ID NO: 6.

In some embodiments, the integrase of the lentiviral vector comprises at least one mutation that retarget viral integration away from gene-dense regions. In some embodiments, the integrase of the lentiviral vector comprises at least one amino acid mutation selected from the group comprising or consisting of S119G and R231G. In some embodiments, the integrase of the lentiviral vector comprises the amino acid mutation S119G, typically the integrase of the lentiviral vector has the amino acid sequence of SEQ ID NO: 5. In some embodiments, the integrase of the lentiviral vector comprises the amino acid mutation R231G, typically the integrase of the lentiviral vector has the amino acid sequence of SEQ ID NO: 9.

In some embodiments, the integrase of the lentiviral vector comprises at least one amino acid mutation at positions selected from the group comprising or consisting of 94, 117, 119, 120, 122, 124, and 231. In some embodiments, the integrase of the lentiviral vector comprises at least one amino acid mutation selected from the group comprising or consisting of G94D, G94E, G94R, G94K, N117D, N117E, N117R, N117K, S119A, S119P, S119T, S119G, S119D, S119E, S119R, S119K, N120D, N120E, N120R, N120K, T122K, T122I, T122V, T122A, T122R, A124D, A124E, A124R, A124K, R231G, R231K, R231D, R231E, and R231S.

In some embodiments, the integrase of the lentiviral vector comprises at least one amino acid mutation at positions selected from the group comprising or consisting of 264, 266, and 273. In some embodiments, the integrase of the lentiviral vector comprises at least one amino acid mutation selected from the group comprising or consisting of K264R, K266R, and K273R.

In some embodiments, the integrase of the lentiviral vector comprises at least one amino acid mutation at positions selected from the group comprising or consisting of 10, 13, 64, 116, 128, 152, 168, and 170. In some embodiments, the integrase of the lentiviral vector comprises at least one amino acid mutation selected from the group comprising or consisting of E10K, E13K, D64A, D64E, D116A, D116E, A128T, E152A, E152D, Q168L, Q168A, and E170G.

In some embodiments, the integrase of the lentiviral vector comprises at least one amino acid mutation at position 168. In some embodiments, the integrase of the lentiviral vector comprises at least one amino acid mutation Q168L or Q168A.

In some embodiments, the integrase of the lentiviral vector comprises at least one mutation selected from the group comprising or consisting of a D116N substitution, a D164N substitution, an insertion of a premature stop codon in its coding sequence, and a deletion of its coding sequence (ΔIN).

In some embodiments, the integrase of the lentiviral vector has an amino acid sequence with SEQ ID NO: 4. In some embodiments, the integrase of the lentiviral vector has an amino acid sequence with SEQ ID NO: 68.

In some embodiments, the nucleic acid sequence encoding the integrase of the lentiviral vector comprises a premature stop codon *(i.e.,* UAA, UAG or UGA). In some embodiments, the resulting integrase is truncated and non-functional. In some embodiments, the resulting integrase is degraded, *e.g.,* by the cell's quality control system.

In some embodiments, the nucleic acid sequence encoding the integrase is deleted from the lentiviral vector's *pol* gene, and is referred to as "ΔIN".

In some embodiments, the lentiviral vector encodes at least one lentiviral polyprotein selected from the group consisting of POL, GAG and ENV. In some embodiments, the lentiviral vector encodes GAG and POL.

In some embodiments, the lentiviral vector comprises a lentiviral vector genome comprising at least one gene selected from the group consisting of *gag* and *pol.* In some embodiments, the lentiviral vector comprises a lentiviral vector genome comprising at least one gene selected from the group consisting of *gag, pol* and *vpr.* In some embodiments, the lentiviral vector comprises a lentiviral vector genome comprising at least one gene selected from the group consisting of *gag, pol, env* and *vpr.* In some embodiments, the lentiviral vector comprises a lentiviral vector genome comprising at least one gene selected from the group consisting of *gag, pol, env, vpr, vpu, vif, nef, tat* and *rev.*

In some embodiments, the lentiviral vector is derived from the human immunodeficiency virus type 1 (HIV-1) or the human immunodeficiency virus type 2 (HIV-2). In some embodiments, the lentiviral vector is derived from HIV-1.

In some embodiments, the lentiviral vector further comprises at least one non-lentiviral protein, or nucleic acid encoding thereof, in particular at least one non-lentiviral surface protein. In some embodiments, the non-lentiviral protein is capable of binding a surface protein of a eukaryotic cell, preferably a mammalian cell, more preferably a human cell.

According to the invention, the lentiviral vector with impaired integration activity optionally comprises an aptamer-binding protein.

In some embodiments, the aptamer-binding protein is fused to the GAG polyprotein of the lentiviral vector. In some embodiments, the C-terminal end of the aptamer-binding protein is fused to the N-terminal end of the GAG polyprotein, optionally through a linker. In some embodiments, the N-terminal end of the aptamer-binding protein is fused to the C-terminal end of the GAG polyprotein, optionally through a linker.

In some embodiments, the aptamer-binding protein is fused to the POL polyprotein of the lentiviral vector. In some embodiments, the C-terminal end of the aptamer-binding protein is fused to the N-terminal end of the POL polyprotein, optionally through a linker. In some embodiments, the N-terminal end of the aptamer-binding protein is fused to the C-terminal end of the POL polyprotein, optionally through a linker.

In some embodiments, the aptamer-binding protein is selected from the group comprising or consisting of MS2 bacteriophage coat protein (MCP), PP7 coat protein (PCP), λN22 peptide and COM.

In some embodiments, the aptamer-binding protein is a MS2 bacteriophage coat protein (MCP).

In some embodiments, MCP has at least 75%, 80%, 85%, 90%, 95%, 96%, 97%, 98%, 99% or 100% identity with SEQ ID NO: 61 (encoded, *e.g.,* by the nucleic acid sequence with SEQ ID NO: 60).

| **SEQ ID NO: 60 - MS2 bacteriophage coat protein - DNA coding sequence** |
|---|
| atggcttcaaactttactcagttcgtgctcgtggacaatggtgggacaggggatgtgacagtggctccttctaatttcgctaatgg ggtggcagagtggatcagctccaactcacggagccaggcctacaaggtgacatgcagcgtcaggcagtctagtgcccagaa gagaaagtataccatcaaggtggaggtccccaaagtggctacccagacagtgggcggagtcgaactgcctgtcgccgcttg gaggtcctacctgaacatggagctcactatcccaattttcgctaccaattctgactgtgaactcatcgtgaaggcaatgcagggg ctcctcaaagacggtaatcctatcccttccgccatcgccgctaactcaggtatctac |

| **SEQ ID NO: 61 - MS2 bacteriophage coat protein** |
|---|
| MASNFTQFVLVDNGGTGDVTVAPSNFANGVAEWISSNSRSQAYKVTCSVRQSS AQKRKYTIKVEVPKVATQTVGGVELPVAAWRSYLNMELTIPIFATNSDCELIVK AMQGLLKDGNPIPSAIAANSGIY |

In some embodiments, the aptamer-binding protein is a MS2 bacteriophage coat protein (MCP), preferably sharing at least 75% identity with SEQ ID NO: 61, and the aptamer is a MS2 RNA tetraloop binding sequence, preferably sharing at least 75% identity with SEQ ID NO: 63.

According to the invention, the composition comprises a nucleic acid encoding a polypeptide or protein comprising an RNA-guided nuclease or nickase.

In some embodiments, the RNA-guided nuclease or nickase is a nuclease protein comprising an active DNA cleavage domain and a guide RNA-binding domain.

In some embodiments, the RNA-guided nuclease or nickase is a Cas protein, including, without limitation, *Streptococcus pyogenes* Cas9 (SpCas9), *Staphylococcus aureus* Cas9 (SaCas9), or *Campylobacter jejuni* Cas9 (CjCas9), or a variant thereof, nickase Cas9 (nCas9), dead Cas9 (dCas9)), Cas12a protein, Cas12b protein, Cas12f protein, Cpf1 protein, or CasX protein, including variants and functional fragments thereof.

In some embodiments, the RNA-guided nuclease or nickase is a Cas9 protein, including variants and functional fragments thereof.

In some embodiments, the RNA-guided nuclease or nickase is a Cas9 protein.

In some embodiments, the Cas9 protein has at least 80%, 90%, 95%, 99% or at least 100% amino acid sequence identity with the sequence of the proteins selected from the group comprising or consisting of *Streptococcus pyogenes* Cas9 (SpCas9) of SEQ ID NO: 34, *Staphylococcus aureus* Cas9 (SaCas9) of SEQ ID NO: 35, Cpf1 of SEQ ID NO: 36, *Campylobacter jejuni* Cas9 (CjCas9) of SEQ ID NO: 37, *Streptococcus pyogenes* Cas9 nickase (nCas9) of SEQ ID NO: 38, and *Staphylococcus aureus* Cas9 nickase of SEQ ID NO: 39.

In some embodiments, the Cas9 protein has at least 80%, 90%, 95%, 99% or at least 100% amino acid sequence identity with the sequence of the proteins selected from the group comprising or consisting of *Staphylococcus aureus* Cas9 (SaCas9) of SEQ ID NO: 34 and *Streptococcus pyogenes* Cas9 (SpCas9) of SEQ ID NO: 35.

In some embodiments, the Cas9 protein is a *Staphylococcus aureus* Cas9 (SaCas9) of SEQ ID NO: 34. In some embodiments, the Cas9 protein is a *Streptococcus pyogenes* Cas9 (SpCas9) of SEQ ID NO: 35.

In some embodiments, the Cas9 variant comprises the amino acid sequence of a Cas9 protein with one or several amino acid substitutions.

In some embodiments, the RNA-guided nuclease or nickase is a variant or a functional fragment of a Cas9 protein.

In some embodiments, the Cas9 variant is humanized Cas9 (hCas9) or a functional fragment thereof. As used herein, the term "humanized Cas9" or "hCas9" refers to a sequence optimized Cas9 protein for human cells. In some embodiments, the hCas9 protein has an amino acid sequence having at least about 70%, at least about 75%, at least about 80%, at least about 85%, at least about 90%, at least about 95%, at least about 96%, at least about 97%, at least about 98%, at least about 99%, or about 100% sequence identity to SEQ ID NO: 40.

In some embodiments, the RNA-guided nuclease or nickase is a CasX protein. In some embodiments, the CasX has an amino acid sequence having at least about 70%, at least about 75%, at least about 80%, at least about 85%, at least about 90%, at least about 95%, at least about 96%, at least about 97%, at least about 98%, at least about 99%, or about 100% sequence identity to SEQ ID NO: 41.

In some embodiments, the RNA-guided nuclease or nickase is a deadCas9 protein. In some embodiments, the deadCas9 has an amino acid sequence having at least about 70%, at least about 75%, at least about 80%, at least about 85%, at least about 90%, at least about 95%, at least about 96%, at least about 97%, at least about 98%, at least about 99%, or about 100% sequence identity to SEQ ID NO: 42.

In some embodiments, the RNA-guided nuclease or nickase is a TnpB (Transposase B from transposon PsiTn554) protein. In some embodiments, the TnpB has an amino acid sequence having at least about 70%, at least about 75%, at least about 80%, at least about 85%, at least about 90%, at least about 95%, at least about 96%, at least about 97%, at least about 98%, at least about 99%, or about 100% sequence identity to SEQ ID NO: 43.

In some embodiments, the RNA-guided nuclease or nickase is a Cas12f protein. In some embodiments, the Cas12f protein is from the bacterium *Acidibacillus sulfuroxidans* (AsCas12f). In some embodiments, the Cas12f has an amino acid sequence having at least about 70%, at least about 75%, at least about 80%, at least about 85%, at least about 90%, at least about 95%, at least about 96%, at least about 97%, at least about 98%, at least about 99%, or about 100% sequence identity to SEQ ID NO: 78.

In some embodiments, the RNA-guided nuclease or nickase is bound to a transposase.

In some embodiments, the RNA-guided nuclease or nickase is bound to a mutated hyperactive PiggyBac transposase (hyPB). In some embodiments, the RNA-guided nuclease or nickase is bound to a hyperactive PiggyBac transposase having at least 75% of amino acid sequence identity with SEQ ID NO: 10.

| **SEQ ID NO: 10 - Hyperactive PiggyBac transposase** |
|---|
| MGSSLDDEHILSALLQSDDELVGEDSDSEVSDHVSEDDVQSDTEEAFIDEVHEV QPTSSGSEILDEQNVIEQPGSSLASNRILTLPQRTIRGKNKHCWSTSKPTRRSRVS ALNIVRSQRGPTRMCRNIYDPLLCFKLFFTDEIISEIVKWTNAEISLKRRESMTSA TFRDTNEDEIYAFFGILVMTAVRKDNHMSTDDLFDRSLSMVYVSVMSRDRFDF LIRCLRMDDKSIRPTLRENDVFTPVRKIWDLFIHQCIQNYTPGAHLTIDEQLLGF RGRCPFRVYIPNKPSKYGIKILMMCDSGTKYMINGMPYLGRGTQTNGVPLGEY YVKELSKPVHGSCRNITCDNWFTSIPLAKNLLQEPYKLTIVGTVRSNKREIPEVL KNSRSRPVGTSMFCFDGPLTLVSYKPKPAKMVYLLSSCDEDASINESTGKPQMV |
| MYYNQTKGGVDTLDQMCSVMTCSRKTNRWPMALLYGMINIACINSFIIYSHNV SSKGEKVQSRKKFMRNLYMGLTSSFMRKRLEAPTLKRYLRDNISNILPKEVPGT SDDSTEEPVMKKRTYCTYCPSKIRRKASASCKKCKKVICREHNIDMCQSCF |

As used herein, the term "mutated hyPB" refers to a transposase comprising one or more amino acid substitutions, typically no more than 1, 2, 3, 4, 5, 6, 7, 8, 9, or 10 amino acid substitutions, as compared to the hyPB of amino acid sequence SEQ ID NO: 10. More specifically, a mutated hyPB comprises (i) one or more amino acid substitutionsto increase excision activity as compared to the hyPB of amino acid sequence SEQ ID NO: 10, and/or (ii) one or more amino acid substitutions to decrease DNA binding activity as compared to the hyPB of amino acid sequence SEQ ID NO: 10. In some embodiments, the mutated hyPB comprises an amino acid sequence at least 85%, at least 90%, at least 95%, at least 96%, at least 97%, at least 98%, or at least 99% identical to the sequence set forth in SEQ ID NO: 10.

In some embodiment, the mutated hyperactive PiggyBac comprises one or more amino acid mutations to increase excision activity.

In some embodiment, the mutated hyperactive Piggybac comprises one or more amino acid mutations to increase excision activity selected among the amino acid mutations within the region defined by the amino acid position numbers [194-200], [214-222], [434-442] or [446-456], for example amino acid substitution at the position D198, D201, R202, M212 and/or S213; said position number corresponding to the amino acid number of unmutated hyperactive Piggybac of SEQ ID NO: 10.

In some embodiment, the mutated hyperactive Piggybac comprises one or more amino acid mutations to increase excision activity selected among the amino acid mutations at positions 450, 560, 564, 573, 589, 592, and/or 594; said position number corresponding to the amino acid number of unmutated hyperactive Piggybac of SEQ ID NO: 10.

In some embodiment, the mutated hyperactive PiggyBac comprises one or more amino acid mutations to increase excision activity selected among the amino acid mutations at position of M194 and/or D450, said position number corresponding to the amino acid number of unmutated hyperactive Piggybac of SEQ ID NO: 10, preferably the amino acid substitution selected among M194V and/or D450N.

In some embodiment, the mutated hyperactive PiggyBac comprises one or more amino acid mutations to decrease DNA binding activity.

In some embodiment, the mutated hyperactive PiggyBac comprises one or more amino acid mutations to decrease DNA binding activity selected among the amino acid mutations at positions 254, 275, 277, 347, 372, 375, and/or 465; said position number corresponding to the amino acid number of unmutated hyperactive Piggybac of SEQ ID NO: 10.

In some embodiment, the mutated hyperactive PiggyBac comprises one or more amino acid mutations to decrease DNA binding activity selected among R275, N347, R372, K375, R376, E377, and E380, said position number corresponding to the amino acid number of unmutated hyperactive Piggybac of SEQ ID NO: 10.

In some embodiment, the mutated hyperactive PiggyBac comprises one or more amino acid mutations to decrease DNA binding activity selected among R372, K375, R376, E377, and E380, said position number corresponding to the amino acid number of unmutated hyperactive Piggybac of SEQ ID NO: 10, preferably selected among the amino acid substitutions R372A, K375A, R376A, E377A, and/or E380A.

In some embodiment, the mutated hyperactive PiggyBac comprises one or more amino acid mutations to decrease DNA binding activity selected among N347, R372, and K375, said position number corresponding to the amino acid number of unmutated hyperactive Piggybac of SEQ ID NO: 10, preferably selected among the amino acid substitutions N347S, N347A, R372A, K375A, more preferably selected among the amino acid substitutions N347S, N347A.

In some embodiment, the mutated hyperactive Piggybac comprises one or more amino acid mutations to increase excision activity, as defined above; and one or more amino acid mutations to decrease DNA binding activity, as defined above.

In some embodiment, the mutated hyperactive Piggybac includes at least one amino acid substitution to increase excision activity at position D450, and at least two amino acid substitutions to decrease DNA binding activity at positions N347, R372 and K375, preferably said mutated transposase of hyperactive Piggybac includes the double mutations N347S and D450N or triple mutations D450N, R372A and K375A, said position number corresponding to the amino acid number of unmutated hyperactive Piggybac of SEQ ID NO: 10. In a more preferred embodiment, the mutated transposase of hyperactive Piggybac includes the double mutations N347S and D450N, said position number corresponding to the amino acid number of unmutated hyperactive Piggybac of SEQ ID NO: 10.

In some embodiment, the mutated hyperactive Piggybac as disclosed in the previous embodiments further comprises at least one mutation in the region defined by the amino acid position numbers [158-169], for example A166S; and/or at least one mutation at position Y527, R518, K525, N463.

Typically, said mutated hyperactive Piggybac comprises an amino acid sequence having at least 85%, at least 90%, at least 95% identity, or 100% identity to mutated hyperactive Piggybac of SEQ ID NO:1.

In some embodiments, said mutated hyperactive Piggybac is a variant of the hyperactive Piggybac of SEQ ID NO: 10 with one or more amino acid substitutions, typically with no more than 1, 2, 3, 4, 5, 6, 7, 8, 9 or 10 amino acid substitutions as compared to SEQ ID NO: 10.

In some embodiments, said mutated hyperactive Piggybac further comprises one or more of the following amino acid mutations at positions 34, 43, 117, 202, 230, 245, 268, 275, 277, 287, 290, 315, 325, 341, 346, 347, 350, 351, 356, 357, 388, 409, 411, 412, 432, 447, 460, 461, 465, 517, 560, 564, 571, 573, 576, 586, 587, 589, 592, and/or 594, the position number corresponding to the amino acid number of the hyperactive PiggyBac sequence (SEQ ID NO: 10).

In some embodiments, said mutated hyperactive PiggyBac comprises the following mutations or combination of mutations: V34M, T43I, Y177H, R202K, S230N, R245A, D268N, K287A, K290A, K287A/K290A, R315A, G325A, R341A, D346N, N347A, N347S, T350A, S351E, S351P, S351A, K356E, N357A, R388A, K409A, A411T, K412A, K432A, D447A, D447N, D450N, R460A, K461A, W465A, S517A, T560A, S564P, S571N, S573A, K576A, H586A, I587A, M589V, S592G, or F594L, D450N/R372A/K375A, R275A/R277A, K409A/K412A, R460A/K461A, R275A/R277A/N347S/K375A/T560A/S573A/M589V/S592G and R245A/R275A/R277A/R372A/W465A.

In some embodiments, said mutated hyperactive PiggyBac comprises the following amino acid substitution or combination of amino acid substitutions:
- R372A/K375A/D450N,
- R372A/K375A/R376A/D450N,
- K375A/R376A/E377A/E380A/D450N,
- R372A/K375A/R376A/E377A/E380A/D450N,
- M194V,
- M194V/R372A/K375A,
- S351A/R372A/K375A/R388A/D450N/W465A/S573A/M589V/S592G/F594L,
- R245A/R275A/R277A/R372A/W465A/M589V,
- R275A/325A/R372A/T560A,
- N347A/D450N,
- N347S/D450N/T560A/S573A/F594L,
- R202K/R275A/N347S/R372A/D450N/T560A/F594L,R275A/N347S/K375A/D45 0N/S592G,
- R275A/N347S/R372A/D450N/T560A/F594L,
- R275A/R277A/N347S/R372A/D450N/T560A/S564P/F594L,
- R245A/N347S/R372A/D450N/T560A/S564P/S573A/S592G,
- R277A/G325A/N347A/K375A/D450N/T560A/S564P/S573A/S592G/F594L,
- V34M/R275A/G325A/N347S/S351A/R372A/K375A/D450N/T560A/S564P,
- G325A/N347S/K375A/D450N/S573A/M589V/S592G,
- S230N/R277A/N347S/K375A/D450N,
- T43I/R372A/K375A/A411T/D450N,
- G325A/N347S/S351A/K375A/D450N/S573A/M589V/S592G, and
- Y177H/R275A/G325A/K375A/D450N/T560A/S564P/S592G,
the position number corresponding to the amino acid number of the hyperactive PiggyBac sequence (SEQ ID NO: 10).

Preferred mutated hyperactive PiggyBac transposases for use according to the present disclosure include mutated hyperactive PiggyBac comprising the following combination of amino acid substitutions:
- R372A/K375A/D450N,
- S351A/R372A/K375A/R388A/D450N/W465A/S573A/M589V/S592G/F594L,
- R245A/R275A/R277A/R372A/W465A/M589V,
- N347A/D450N,
- N347S/D450N/T560A/S573A/F594L,
- R202K/R275A/N347S/R372A/D450N/T560A/F594L,
- R275A/N347S/K375A/D450N/S592G,
- R275A/N347S/R372A/D450N/T560A/F594L,
- R275A/R277A/N347S/R372A/D450N/T560A/S564P/F594L,
- R245A/N347S/R372A/D450N/T560A/S564P/S573A/S592G,
- R277A/G325A/N347A/K375A/D450N/T560A/S564P/S573A/S592G/F594L,
- V34M/R275A/G325A/N347S/S351A/R372A/K375A/D450N/T560A/S564P,
- G325A/N347S/K375A/D450N/S573A/M589V/S592G,
- S230N/R277A/N347S/K375A/D450N,
- T43I/R372A/K375A/A411T/D450N,
- G325A/N347S/S351A/K375A/D450N/S573A/M589V/S592G,
- Y177H/R275A/G325A/K375A/D450N/T560A/S564P/S592G, and
- R275A/325A/R372A/T560A,
the position number corresponding to the amino acid number of the hyperactive PiggyBac sequence (SEQ ID NO: 10).

In some embodiments, said mutated hyperactive PiggyBac comprises the following amino acid substitution or combination of amino acid substitutions:
- R245A/R275A/R277A/R372A/W465A/M589V,
- R275A/325A/R372A/T560A,
- N347A/D450N,
- N347S/D450N/T560A/S573A/F594L,
- R202K/R275A/N347S/R372A/D450N/T560A/F594L,
- R275A/N347S/K375A/D450N/S592G,
- R275A/N347S/R372A/D450N/T560A/F594L,
- R275A/R277A/N347S/R372A/D450N/T560A/S564P/F594L,
- R245A/N347S/R372A/D450N/T560A/S564P/S573A/S592G,
- R277A/G325A/N347A/K375A/D450N/T560A/S564P/S573A/S592G/F594L,
- G325A/N347S/K375A/D450N/S573A/M589V/S592G,
- S230N/R277A/N347S/K375A/D450N,
- G325A/N347S/S351A/K375A/D450N/S573A/M589V/S592G, and
- Y177H/R275A/G325A/K375A/D450N/T560A/S564P/S592G,
the position number corresponding to the amino acid number of the hyperactive PiggyBac sequence (SEQ ID NO: 10).

In a more preferred embodiment, said mutated hyperactive PiggyBac comprising the following combination of amino acid substitutions:
- N347A/D450N,
- N347S/D450N/T560A/S573A/F594L,
- R202K/R275A/N347S/R372A/D450N/T560A/F594L,
- R275A/N347S/K375A/D450N/S592G,
- R275A/N347S/R372A/D450N/T560A/F594L,
- R275A/R277A/N347S/R372A/D450N/T560A/S564P/F594L,
- R245A/N347S/R372A/D450N/T560A/S564P/S573A/S592G,
- R277A/G325A/N347A/K375A/D450N/T560A/S564P/S573A/S592G/F594L,
- G325A/N347S/K375A/D450N/S573A/M589V/S592G,
- S230N/R277A/N347S/K375A/D450N,
- G325A/N347S/S351A/K375A/D450N/S573A/M589V/S592G,
- Y177H/R275A/G325A/K375A/D450N/T560A/S564P/S592G,
the position number corresponding to the amino acid number of the hyperactive PiggyBac sequence (SEQ ID NO: 10).

In some embodiments, said mutated transposase comprises the following combination of amino acid substitutions: R372A/K375A/D450N, said position numbers corresponding to the amino acid numbers of unmutated hyperactive PiggyBac of SEQ ID NO: 10. In some embodiment, said mutated transposase has an amino acid sequence of SEQ ID NO: 11.

In some embodiments, said mutated transposase has an amino acid sequence selected among any of SEQ ID NO: 12-33.

In some embodiments, said mutated transposase has an amino acid sequence selected among any of SEQ ID NO: 12 to SEQ ID NO: 20.

In some embodiments, said mutated transposase has an amino acid sequence selected among any of SEQ ID NO: 21 to SEQ ID NO: 33.

In some embodiments, the mutated transposase can comprise one or more mutations relative to hyPB that are involved in the conserved catalytic triad, *e.g.,* at amino acid 268 and/or 346 (e.g., D268N and/or D346N) corresponding to the amino acid numbering of SEQ ID NO: 10.

In some embodiments, the mutated transposase can comprise one or more mutations relative to hyPB that are critical for excision, *e.g*., at amino acid 287, 287/290 and/or 460/461 *(e.g.,* K287A, K287A/K290A, and/or R460A/K461A) corresponding to the amino acid numbering of SEQ ID NO: 10.

In some embodiments, the mutated transposase can comprise one or more mutations relative to hyPB that are involved in target joining, *e.g.,* at amino acid 351, 356, and/or 379 (*e.g.,* S351E, S351P, S351A, and/or K356E) corresponding to the amino acid numbering of SEQ ID NO: 10.

In some embodiments, the mutated transposase can comprise one or more mutations relative to hyPB that are critical for integration, *e.g*., at amino acid 560, 564, 571, 573, 589, 592, and/or 594 *(e.g.,* T560A, S564P, S571N, S573A, M589V, S592G, and/or F594L) corresponding to the amino acid numbering of SEQ ID NO: 10.

In some embodiments, the mutated transposase can comprise one or more mutations relative to hyPB that are involved in alignment, *e.g*., at amino acid 325, 347, 350, 357 and/or 465 (*e.g.,* G325A, N347A, N347S, T350A and/or W465A) corresponding to the amino acid numbering of SEQ ID NO: 10.

In some embodiments, the mutated transposase can comprise one or more mutations relative to hyPB that are well conserved, *e.g*., at amino acid 576 and/or 587 (*e.g.,* K576A and/or I587A) corresponding to the amino acid numbering of SEQ ID NO: 10.

In some embodiments, the mutated transposase can comprise one or more mutations relative to hyPB that are involved in Zn²⁺ binding, *e.g.,* 586 (*e.g.,* H586A) corresponding to the amino acid numbering of SEQ ID NO: 10.

In some embodiments, the programmable transposase can comprise one or more mutations relative to hyPB that are involved in integration, *e.g*., 315, 341, 372, and/or 375 *(e.g.,* R315A, R341A, R372A, and/or K375A) corresponding to the amino acid numbering of SEQ ID NO: 10.

In some embodiments, the mutated hyperactive PiggyBac comprises an amino acid sequence at least 85%, at least 90%, at least 95%, at least 96%, at least 97%, at least 98%, or at least 99% identical to the sequence set forth in SEQ ID NO: 10. In some embodiments, the mutated hyperactive PiggyBac is selected for its high specificity of DNA integration into a genome compared to hyperactive PiggyBac. In some embodiments, the mutated hyperactive PiggyBac comprises an amino acid sequence having one or more of the modifications disclosed herein relative to SEQ ID NO: 10, and retains at least 80%, at least 85%, at least 90%, at least 95%, at least 96%, at least 97%, at least 98%, or at least 99% identical to the sequence set forth in SEQ ID NO: 12 to SEQ ID NO: 33.

In some embodiments, the mutated hyperactive PiggyBac transposase can comprise a mutation of one or more of amino acids selected from amino acid: 245, 275, 277, 325, 347, 351, 372, 375, 388, 450, 465, 560, 564, 573, 589, 592, 594 corresponding to the amino acid numbering of SEQ ID NO: 10.

In some embodiments, the mutated hyperactive PiggyBac transposase mutation can comprise one or more of the amino acid modifications selected from: R245A, R275A, R277A, R275A/R277A, G325A, N347A, N347S, S351E, S351P, S351A, R372A, K375A, R388A, D450N, W465A, T560A, S564P, S573A, M589V, S592G, or F594L corresponding to the amino acid numbering of SEQ ID NO: 10.

In an embodiment, the mutated hyperactive PiggyBac transposase comprises the amino acid modification D450N corresponding to the amino acid numbering of SEQ ID NO: 10.

In an embodiment, the mutated hyperactive PiggyBac transposase comprises the amino acid modifications R245A and D450, corresponding to the amino acid numbering of SEQ ID NO: 10.

In an embodiment, the mutated hyperactive PiggyBac transposase comprises the amino acid modifications R245A, G325A, and S573P, corresponding to the amino acid numbering of SEQ ID NO: 10.

In an embodiment, the mutated hyperactive PiggyBac transposase comprises the amino acid modifications R245A, G325A, D450 and S573P, corresponding to the amino acid numbering of SEQ ID NO: 10.

In an embodiment, the mutated hyperactive PiggyBac transposase comprises the amino acid modification N347S or N347A, corresponding to the amino acid numbering of SEQ ID NO: 10.

In an embodiment, the mutated hyperactive PiggyBac transposase comprises the amino acid modifications N347S and D450N, corresponding to the amino acid numbering of SEQ ID NO: 10.

In another, the mutated hyperactive PiggyBac transposase comprises the amino acid modifications N347A and D450N, corresponding to the amino acid numbering of SEQ ID NO: 10. In some embodiments, this mutated hyperactive PiggyBac transposase comprises the amino acid sequence of SEQ ID NO: 21.

In some embodiments, the mutated hyperactive PiggyBac transposase comprises the amino acid sequence SEQ ID NO: 10, wherein:
- amino acid residue at position 34 is V or M,
- amino acid residue at position 43 is T or I,
- amino acid residue at position 177 is Y or H,
- amino acid residue at position 202 is R or K,
- amino acid residue at position 230 is S or N,
- amino acid residue at position 245 is A,
- amino acid residue at position 268 is D or N,
- amino acid residue at position 277 is R or A,
- amino acid residue at position 275 is R or A,
- amino acid residue at position 277 is R or A,
- amino acid residue at position 325 is A or G,
- amino acid residue at position 347 is S, or A,
- amino acid residue at position 351 is E, P or A,
- amino acid residue at position 372 is R or A,
- amino acid residue at position 375 is K or A,
- amino acid residue at position 388 is R or A,
- amino acid residue at position 409 is K or A,
- amino acid residue at position 411 is A or T,
- amino acid residue at position 412 is K or A,
- amino acid residue at position 450 is D or N,
- amino acid residue at position 460 is R or A,
- amino acid residue at position 465 is W or A,
- amino acid residue at position 517 is S or A,
- amino acid residue at position 560 is T or A,
- amino acid residue at position 564 is P or S,
- amino acid residue at position 571 is S or N,
- amino acid residue at position 573 is S or A,
- amino acid residue at position 576 is K or A,
- amino acid residue at position 586 is H or A,
- amino acid residue at position 587 is I or A,
- amino acid residue at position 589 is M or V,
- amino acid residue at position 592 is G or S, and/or,
- amino acid residue at position 594 is L or F.

In some embodiments, the mutated hyperactive PiggyBac transposase comprises or consists of an amino acid sequence having at least 75%, 80%, 85%, 90%, 95%, 96%, 97%, 98%, 99%, 99.5%, 99.9% or 100% sequence identity with the amino acid sequence selected from the group comprising or consisting of SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32 and SEQ ID NO: 33.

In some embodiments, the mutated transposase is not a Himar1C9 mutant.

In some embodiments, the RNA-guided nuclease or nickase and the hyPB are bound together in the form of a fusion protein. In some embodiments, the RNA-guided nuclease or nickase and the hyPB are comprised in a fusion protein. In some embodiments, the fusion protein further comprises a linker.

In some embodiments, the RNA-guided nuclease/nickase-hyPB fusion protein comprises or consists of:
(i) a first protein comprising or consisting of an RNA-guided nuclease or nickase, as described hereinabove,
(ii) optionally, a linker, and
(iii) a second protein comprising or consisting of a transposase, said transposase being a mutated hyPB comprising one or more amino acid mutations as compared to hyPB of SEQ ID NO: 10, as described hereinabove.

In some embodiments, the RNA-guided nuclease or nickase comprised in the RNA-guided nuclease/nickase-hyPB fusion protein is a Cas9 protein as described hereinabove.

In some embodiments, the RNA-guided nuclease/nickase-hyPB fusion protein comprises or consists of:
(i) a first protein comprising or consisting of a Cas9 protein or a variant thereof, as described hereinabove,
(ii) optionally, a linker, and
(iii) second protein comprising or consisting of a transposase, said transposase being a mutated hyPB comprising one or more amino acid mutations as compared to hyPB of SEQ ID NO: 10, as described hereinabove.

In some embodiment, the first protein and the second protein can be oriented in the fusion protein in either order.

In some embodiment, the N-terminal end of the RNA-guided nuclease or nickase is fused to the C-terminal end of the hyPB, either directly or indirectly via a linker. In another embodiment, the N-terminal end of the hyPB is fused to the C-terminal end of the RNA-guided nuclease or nickase, either directly or indirectly via a linker.

In some embodiment, the RNA-guided nuclease or nickase (e.g., Cas9 protein) is fused to a lentiviral protein, preferably a structural lentiviral protein (e.g., capsid or matrix protein), in order to facilitate its packaging in a lentiviral particle.

In some embodiment, the RNA-guided nuclease or nickase is bound to a Viral Protein R (VPR). In some embodiments, VPR has an amino acid sequence having at least 75%, 80%, 85%, 90%, 95%, 96%, 97%, 98%, 99%, 99.5%, 99.9% or 100% sequence identity with SEQ ID NO: 67.

| **SEQ ID NO: 67** |
|---|
| MEQAPEDQGPQREPYNEWTLELLEELKREAVRHFPRPWLHGLGQHIYETYGDT WTGVEAIIRILQRLLFVHFRIGCQHSRIGILRQRRARNGASRS |

In some embodiments, the VPR-RNA-guided nuclease/nickase fusion protein comprises or consists of:
(i) a first protein comprising or consisting of VPR, or variants thereof,
(ii) optionally, a linker, and
(iii) a second protein comprising or consisting of an RNA-guided nuclease or nickase, as described hereinabove.

In some embodiments, the RNA-guided nuclease or nickase and VPR are bound together in the form of a fusion protein. In some embodiments, the RNA-guided nuclease or nickase comprised in the fusion protein is a Cas9 protein as described hereinabove.

In some embodiments, the VPR-Cas9 fusion protein comprises or consists of:
(i) a first protein comprising or consisting of VPR, or variants thereof,
(ii) optionally, a linker, and
(iii) a second protein comprising or consisting of a Cas9 protein or a variant thereof, as described hereinabove.

In some embodiments, the first protein and the second protein can be oriented in the fusion protein in either order.

In some embodiments, the N-terminal end of the Cas9 is fused to the C-terminal end of VPR, either directly or indirectly via a linker. In some embodiments, the VPR-Cas9 fusion protein has at least 75%, 80%, 85%, 90%, 95%, 96%, 97%, 98%, 99%, 99.5%, 99.9% or 100% sequence identity with SEQ ID NO: 66.

In some embodiments, the C-terminal end of the Cas9 is fused to the N-terminal end of VPR, either directly or indirectly via a linker.

In some embodiments, the VPR-Cas9 fusion protein is further fused to a RNA-guided nuclease or nickase as described hereinabove.

In some embodiment, the RNA-guided nuclease or nickase is bound to Lens Epithelium-Derived Growth Factor (LEDGF), interchangeably referred to as P75.

In some embodiments, the P75-RNA-guided nuclease/nickase fusion protein comprises or consists of:
(i) a first protein comprising or consisting of P75, or variants thereof,
(ii) optionally, a linker, and
(iii) a second protein comprising or consisting of an RNA-guided nuclease or nickase, as described hereinabove.

In some embodiments, the RNA-guided nuclease or nickase and P75 are bound together in the form of a fusion protein. In some embodiments, the RNA-guided nuclease or nickase comprised in the fusion protein is a Cas9 protein as described hereinabove.

In some embodiments, the P75-Cas9 fusion protein comprises or consists of:
(i) a first protein comprising or consisting of P75, or variants thereof,
(ii) optionally, a linker, and
(iii) a second protein comprising or consisting of a Cas9 protein or a variant thereof, as described hereinabove.

In some embodiments, the first protein and the second protein can be oriented in the fusion protein in either order.

In some embodiments, the N-terminal end of the Cas9 is fused to the C-terminal end of P75, either directly or indirectly via a linker. In another embodiment, the C-terminal end of the Cas9 is fused to the N-terminal end of P75, either directly or indirectly via a linker.

In some embodiments, the P75-Cas9 fusion protein has an amino acid-sequence at least 75%, 80%, 85%, 90%, 95%, 99% or 100% identical to any one of SEQ ID NO: 69 to 73. In some embodiments, the P75-Cas9 fusion protein has the amino acid-sequence selected from the group comprising or consisting of SEQ ID NO: 69 to 73. In some embodiments, the P75-Cas9 fusion protein has the amino acid-sequence of SEQ ID NO: 69. In some embodiments, the P75-Cas9 fusion protein has the amino acid-sequence of SEQ ID NO: 70. In some embodiments, the P75-Cas9 fusion protein has the amino acid-sequence of SEQ ID NO: 71. In some embodiments, the P75-Cas9 fusion protein has the amino acid-sequence of SEQ ID NO: 72. In some embodiments, the P75-Cas9 fusion protein has the amino acid-sequence of SEQ ID NO: 73.

In some embodiments, the P75-Cas9 fusion protein is further fused to a RNA-guided nuclease or nickase as described hereinabove.

In some embodiments, the RNA-guided nuclease or nickase is not fused to an integrase. In some embodiments, the RNA-guided nuclease or nickase is not fused to the integrase of the lentiviral vector of (i). In some embodiments, the RNA-guided nuclease or nickase is not fused to the integrase, when present, of the fusion protein encoded by the nucleic acid of (v).

According to the invention, the composition comprises a nucleic acid encoding a guide RNA (gRNA) fused to at least one aptamer.

As used herein, the term "at least one" means 1, 2, 3, 4, 5, 6, 7, 8, 9, 10 or more.

In some embodiments, the aptamer is an RNA sequence comprising a tetraloop. The term "tetraloop" is used interchangeably with the terms "stem loop" and "hairpin loop".

In some embodiments, the aptamer-binding protein described herein is capable of binding to the at least one aptamer.

In some embodiments, the at least one tetraloop is a MS2 RNA tetraloop binding sequence. In some embodiments, the at least one MS2 RNA tetraloop binding sequence has at least 75%, 80%, 85%, 90%, 95%, 96%, 97%, 98%, 99% or 100% identity with SEQ ID NO: 63 (encoded, *e.g.,* by the DNA sequence of SEQ ID NO: 62).

| **SEQ ID NO: 62 - MS2 RNA tetraloop binding sequence - DNA coding sequence** |
|---|
| gggggccactagggacaggatgttttagagctaggccaacatgaggatcacccatgtctgcagggcctagcaagttaaaataa ggctagtccgttatcaacttggccaacatgaggatcacccatgtctgcagggccaagtggcaccgagtcggtgcttttttt |

| **SEQ ID NO: 63 - MS2 RNA tetraloop binding sequence** |
|---|
| ggggccacuagggacaggauguuuuagagcuaggccaacaugaggaucacccaugucugcagggccuagcaagu uaaaauaaggcuaguccguuaucaacuuggccaacaugaggaucacccaugucugcagggccaaguggcaccgag ucggugcuuuuuu |

In some embodiments, the gRNA is capable of forming a complex with an RNA-guide nuclease or nickase as described hereinabove, *e.g*., a Cas9 protein or fusion protein comprising a Cas9.

In some embodiments, the gRNA is capable of targeting the RNA-guide nuclease or nickase as described hereinabove to a specific sequence or region of the genome of a cell, preferably a mammalian cell, more preferably a human cell. In some embodiments, the specific sequence targeted by the gRNA is adjacent to a protospacer adjacent motif (PAM) specific for the Cas9 protein.

In some embodiments, the specific sequence targeted by the gRNA may be within a safe harbor locus in a cell's genome. A **"safe harbor locus"** refers to a region of a cell's genome, where the integrated material can be adequately expressed without perturbing endogenous gene structure or function. Safe harbor loci include, but are not limited to, AAVS1 (intron 1 of PPP1R12C), HPRT, HI 1, hRosa26, albumin and F-A region. The safe harbor loci may be exons or introns of ubiquitously expressed genes and/or genes with tissue specific expression (*e.g*., muscle). Safe harbor loci can be selected from the group consisting of: exon 1, intron 1 or exon 2 of PPP1R12C, exon 1, intron 1 or exon 2 of HPRT, and exon 1, intron 1 or exon 2 of hRosa26, intron 1 of the *albumin* gene. A safe harbor locus may also include a region of the genome devoid of endogenous genes and with open chromatin that allows for the expression of the inserted transgene without perturbing the genome structure or function.

In some embodiments, the specific sequence targeted by the gRNA may be within or in close proximity to a gene in a cell's genome. For instance, it is conceivable that the specific sequence targeted by the gRNA be within or in close proximity to a gene which requires gene repair or genetic modification (by integration of an exogenous copy of the gene, brought by the nucleic acid encoding the transgene of interest of (iv)).

According to the invention, the composition comprises a nucleic acid encoding a transgene of interest.

As used herein, the term **"transgene of interest"** refers to an exogenous nucleic acid sequence to be inserted in the genome of a cell, preferably a eukaryotic cell, more preferably a mammalian cell, that comprises at least one open reading frame. Typically, the transgene of interest is a coding sequence, *i.e.,* it encodes at least one protein or polypeptide. However, the transgene of interest may also be a non-coding sequence.

In some embodiments, the transgene of interest comprises at least one gene and at least one non-coding sequence, *e.g*., regulatory elements, or promoters.

In some embodiments, the transgene of interest comprises at least one gene. In some embodiment, the at least one gene is absent from the genome of the cell. In some embodiment, the at least one gene is an allele of a gene of the genome of the cell, wherein this allele is not endogenous to the genome of the cell.

In some embodiments, the transgene of interest comprises at least one gene from the same species than the cell's. In another embodiment, the transgene of interest comprises at least one gene from a different species than the cell's.

In some embodiments, the transgene of interest has a length of at least 500 base pairs (bp). As use herein, at least 500 means 500, 600, 700, 800, 900, 1,000, 10,000, 20,000 bp or more.

In some embodiments, the transgene of interest is a DNA molecule, an RNA molecule, or combinations thereof. In some embodiments, the transgene of interest is a DNA molecule. In some embodiments, the transgene of interest is an RNA molecule.

In some embodiments, the transgene of interest may be flanked by inverted terminal repeat (ITR) sequences. This is particularly desirable when the RNA-guided nuclease or nickase is bound to a transposase, as described above.

According to the invention, the composition optionally comprises a nucleic acid encoding a fusion protein comprising (1) a first protein comprising or consisting of a GAG polyprotein, (2) a second protein comprising or consisting of an aptamer-binding protein, and optionally (3) a third protein comprising or consisting of a POL polyprotein.

As used herein, the term **"GAG"** refers to a retroviral polyprotein. The GAG polyprotein is typically post-translationally processed by the virus' protease (PR) into MA (matrix), CA (capsid), and NC (nucleocapsid) parts, and sometimes more. For instance, the GAG polyprotein of the HIV-1 virus is post-translationally processed into matrix protein p17, capsid protein p24, nucleocapsid protein p7 and p6-gag. In some embodiments, the fusion protein comprises a protein comprising or consisting of the HIV-1 GAG polyprotein.

As used herein, the term **"POL"** refers to a retroviral polyprotein. The POL polyprotein is typically post-translationally processed by the virus' protease (PR) into PR (protease), RT (reverse transcriptase), p15 (ribonuclease H), and INT (integrase) parts. The protease is itself released by autocatalytic cleavage. In some embodiments, the fusion protein comprises a protein comprising or consisting of the HIV-1 POL polyprotein.

In some embodiments, the fusion protein comprises a POL polyprotein and thus the composition may comprise nucleic acid molecules encoding two distinct integrases: one integrase with impaired integration activity in the lentiviral vector of (i); and one integrase in the fusion protein of (v).

In some embodiments, the aptamer-binding protein is fused to the GAG polyprotein. In some embodiments, the C-terminal end of the aptamer-binding protein is fused to the N-terminal end of the GAG polyprotein, optionally through a linker. In some embodiments, the N-terminal end of the aptamer-binding protein is fused to the C-terminal end of the GAG polyprotein, optionally through a linker.

In some embodiments, the C-terminal end of the aptamer-binding protein is fused to the N-terminal end of the polyprotein GAG, optionally through a linker, typically the fusion protein has the amino acid sequence of SEQ ID NO: 64.

| **SEQ ID NO 64** |
|---|
| MASNFTQFVLVDNGGTGDVTVAPSNFANGVAEWISSNSRSQAYKVTCSVRQSS AQKRKYTIKVEVPKVATQTVGGVELPVAAWRSYLNMELTIPIFATNSDCELIVK AMQGLLKDGNPIPSAIAANSGIYSQNYPIVQSGLRSRAQASNSAVDGTAGPGSM DSGRDFLTLHGLQDDEDLQALLKGSQLLKVKSSSWRRERFYKLQEDCKTIWQE SRKVMRTPESQLFSIEDIQEVRMGHRTEGLEKFARDVPEDRCFSIVFKDQRNTL DLIAPSPADAQHWVLGLHKIIHHSGSMDQRQKLQHWIHSCLRKADKNKDNKM SFKELQNFLKELNIQPRAEFTLAARASVLSGGELDRWEKIRLRPGGKKKYKLKH IVWASRELERFAVNPGLLETSEGCRQILGQLQPSLQTGSEELRSLYNTVATLYCV HQRIEIKDTKEALDKIEEEQNKSKKKAQQAAADTGHSSQVSQNYPIVQNIQGQ MVHQAISPRTLNAWVKVVEEKAFSPEVIPMFSALSEGATPQDLNTMLNTVGGH QAAMQMLKETINEEAAEWDRVHPVHAGPIAPGQMREPRGSDIAGTTSTLQEQI GWMTNNPPIPVGEIYKRWIILGLNKIVRMYSPTSILDIRQGPKEPFRDYVDRFYK TLRAEQASQEVKNWMTETLLVQNANPDCKTILKALGPAATLEEMMTACQGVG GPGHKARVLAEAMSQVTNTATIMMQRGNFRNQRKMVKCFNCGKEGHTARNC RAPRKKGCWKCGKEGHQMKDCTERQANFLGKIWPSYKGRPGNFLQSRPEPTA PPFLQSRPEPTAPPEESFRSGVETTTPPQKQEPIDKELYPLTSLRSLFGNDPSSQ |

In some embodiments, the aptamer-binding protein is fused to the POL polyprotein, if present. In some embodiments, the C-terminal end of the aptamer-binding protein is fused to the N-terminal end of the POL polyprotein, preferably of the GAG-POL polyprotein, optionally through a linker. In some embodiments, the N-terminal end of the aptamer-binding protein is fused to the C-terminal end of the POL polyprotein, preferably of the GAG-POL polyprotein, optionally through a linker.

In some embodiments, the C-terminal end of the aptamer-binding protein is fused to the N-terminal end of the polyprotein GAG-POL, optionally through a linker, typically the fusion protein has the amino acid sequence of SEQ ID NO: 65.

| **SEQ ID NO: 65** |
|---|
| MASNFTQFVLVDNGGTGDVTVAPSNFANGVAEWISSNSRSQAYKVTCSVRQSS AQKRKYTIKVEVPKVATQTVGGVELPVAAWRSYLNMELTIPIFATNSDCELIVK AMQGLLKDGNPIPSAIAANSGIYSQNYPIVQSGLRSRAQASNSAVDGTAGPGSM |
| DSGRDFLTLHGLQDDEDLQALLKGSQLLKVKSSSWRRERFYKLQEDCKTIWQE SRKVMRTPESQLFSIEDIQEVRMGHRTEGLEKFARDVPEDRCFSIVFKDQRNTL DLIAPSPADAQHWVLGLHKIIHHSGSMDQRQKLQHWIHSCLRKADKNKDNKM SFKELQNFLKELNIQPRAEFTLAARASVLSGGELDRWEKIRLRPGGKKKYKLKH IVWASRELERFAVNPGLLETSEGCRQILGQLQPSLQTGSEELRSLYNTVATLYCV HQRIEIKDTKEALDKIEEEQNKSKKKAQQAADTGHSSQVSQNYPIVQNIQGQ MVHQAISPRTLNAWVKVVEEKAFSPEVIPMFSALSEGATPQDLNTMLNTVGGH QAAMQMLKETINEEAAEWDRVHPVHAGPIAPGQMREPRGSDIAGTTSTLQEQI GWMTNNPPIPVGEIYKRWIILGLNKIVRMYSPTSILDIRQGPKEPFRDYVDRFYK TLRAEQASQEVKNWMTETLLVQNANPDCKTILKALGPAATLEEMMTACQGVG GPGHKARVLAEAMSQVTNTATIMMQRGNFRNQRKMVKCFNCGKEGHTARNC RAPRKKGCWKCGKEGHQMKDCTERQANFLGKIWPSYKGRPGNFLQSRPEPTA PPFLQSRPEPTAPPEESFRSGVETTTPPQKQEPIDKELYPLTSLRSLFGNDPSSQRS VASRRPCWTPAPTTPCWRRACPAGGSPRSAASAASSRGSTTRSSRSAATRPSAP CWWDLHLTSSAGTCPRSAAPTSPSAPSRPCPSSPAWTALRSSGPPRRRSRPWWR SAPRWRRRARSARSAPRTPTTPPCSPSRRRTAPSGGSWWTSGSTRGPRTSGRCS WASPTPPARRRRAPCWTWATPTSACPWTRTSGSIPPSPSPASTTRPPASATSTTC CPRAGRAAPPSSRAAQRSWSPSRSRTPTSSISTWTTCTWAATWRSASTGPRSRS GSTCGGAPPPTRSTRRSPHSCGWATSCTPTSGPCSPSCCPRRTAGPTTFRSWWAS TGPARSTLASRGSCASCGAQRLPRSPPRRPSWSWPRTGRSRSPCTACTTTPARTS PRSRSRARASGPTRSTRSPSRTRPASTPACAAPTPTTSSPRPCRRSPPRASSGARL LSSSCPSRRRPGRPGGPSTGRPPGFPSGSSTPLPWSCGISWRRSPSWAPRPSTWTA PPTGRPSWARPATPTRAARRWCPPTPPTRRPSCRLSTWPCRTQAWRTSPTASTP WASSRPSPTRARASWTRSSSSSRRRRCTWPGCPPTRASAATSRWTSWAPASGRS CSWTASTRPRTSTRSTTATGGLWLATSTCLPWWLRRSWPAATSASRARPCTAR WTAAPASGSWIAPTWRARSWWPCTWPPATSRPRSPPRPARRPPTSCSWPAAGP RPSTPTTAATSPAPPRLPAGGPASSRSSASPTTPSLRAWWRATRSRRSSARGTRP STRPPCRWPCSSTTSRGRAASAATAPARGSWTSSPPTSRPRSCRSRSPRSRTSGC TTGTAGTLCGRAPPSCCGRARAPWSRTTATSRWCPGGRPRSSGTTASRWPATT AWPPGRTRT |

Aptamer-binding proteins have been described above, which description applies here *mutatis mutandis.*

In some embodiments, when the fusion protein comprises a third protein comprising or consisting of a POL polyprotein, the integrase of the POL polyprotein may have impaired integration activity.

Mutations, including substitutions and deletions, to impair the integration activity of an integrase, have been described above, which description applies here *mutatis mutandis.*

In some embodiments, the integrase of the POL polyprotein may comprise the same or different mutation(s) than the integrase of the lentiviral vector of (i).

In some embodiments, the fusion protein comprises one or several linkers.

In some embodiments, the linker is a peptidic linker. In one embodiment, the peptidic linker is selected from the group comprising or consisting of (GGS)ₙ, (GGGGS)ₙ, (G)n, (EAAAK)n, XTEN linkers, and (XP)n motif, and combinations of any of any of these, wherein n is independently an integer between 1 and 50.

In some embodiments, the linker is 1- to 24-amino acids long, or is encoded by a nucleic acid sequence that is 3- to 72- nucleotides long. In one embodiment, the linker is 1- to 12-amino acids long, or is encoded by a nucleic acid sequence that is 3- to 36- nucleotides long. In one embodiment, the linker is 1- to 6-amino acids long, or is encoded by a nucleic acid sequence that is 3- to 18- nucleotides long.

In some embodiments, the linker is a XTEN linker or a (GGS)ₙ linker.

In some embodiments, the linker is selected among the linkers shown in **Table 1.**

**Table 1: Linkers**

| **Linker** | **Nucleic acid sequence (SEQ ID NO)** | **Amino acid sequence (SEQ ID NO)** |
|---|---|---|
| GGSx3 | ggtggatctggcggtggatctggtggcggt | GGSGGGSGGG |
| | (SEQ ID NO: 44) | (SEQ ID NO: 45) |
| GGS4x | ggagggagtggtgggtccggtggtagtggcggatcc | GGSGGSGGSGGS |
| | (SEQ ID NO: 46) | (SEQ ID NO: 47) |
| GGS5x | ggaggctccggtgggtctggtgggagcggtggtagtggcgg atcc | GGSGGSGGSGGSGGS |
| | (SEQ ID NO: 48) | (SEQ ID NO: 49) |
| GGS6x | ggaggcagtggtgggagcggtggttccgggggtagtggtgg ttccgggggatcc | GGSGGSGGSGGSGGSGGS |
| | (SEQ ID NO: 50) | (SEQ ID NO: 51) |
| GGS7x | ggaggttctggaggctccggtgggtccgggggaagtgggg ggtcaggcggatcaggaggatcc | GGSGGSGGSGGSGGSGGSG GS |
| | (SEQ ID NO: 52) | (SEQ ID NO: 53) |
| GGS8x | ggaggtagcggaggttccggagggagcggcgggagtggg ggaagcgggggaagtggaggatccgggggaggatcc | GGSGGSGGSGGSGGSGGSG GS |
| | (SEQ ID NO: 54) | (SEQ ID NO: 55) |
| Linker XTEN | tccggtagcgaaacaccggggacttcagaatcggccacccc ggagtct | SGSETPGTSESATPES |
| | (SEQ ID NO: 56) | (SEQ ID NO: 57) |
| Linker B | ggaagcgccggtagtgcggctgggtctggcgagttc | GSAGSAAGSGEF |
| | (SEQ ID NO: 58) | (SEQ ID NO: 59) |

In some embodiments, the linker comprises an amino acid sequence selected from the group comprising or consisting of SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, or any combination thereof; respectively encoded by the exemplary nucleic acid sequence of SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58.

In some embodiments, the linker comprises or consists of the amino acid sequence of SEQ ID NO: 45; encoded by the exemplary nucleic acid sequence of SEQ ID NO: 44.

In some embodiments, the aptamer-binding protein is fused to the mutated integrase in any order. In some embodiments, the aptamer-binding protein is fused to the mutated integrase through the N-terminal end. In some embodiments, the aptamer-binding protein is fused to the integrase through the C-terminal end. In some embodiments, the N-terminal end of the aptamer-binding protein is fused to the C-terminal end of the mutated integrase, optionally through a linker. In some embodiments, the C-terminal end of the aptamer-binding protein is fused to the N-terminal end of the mutated integrase, optionally through a linker.

In some embodiments, the fusion protein further comprises a Viral Protein R (VPR). In some embodiments, the aptamer-binding protein is fused to the integrase of the POL polyprotein, and the integrase of the POL polyprotein is further fused to VPR. In some embodiments, VPR has an amino acid sequence having at least 75%, 80%, 85%, 90%, 95%, 96%, 97%, 98%, 99%, 99.5%, 99.9% or 100% sequence identity with SEQ ID NO: 67. In some embodiments, the aptamer-binding protein is fused to the GAG polyprotein, and the GAG polyprotein is further fused to VPR. In some embodiments, the aptamer-binding protein is fused to the GAG-POL polyprotein, and the GAG-POL polyprotein is further fused to VPR.

In some embodiments, the N-terminal end of the integrase of the POL polyprotein is fused to the C-terminal end of VPR, optionally through a linker, and the N-terminal end of the aptamer-binding protein is fused to the C-terminal end of the integrase of the POL polyprotein, optionally through a linker. In some embodiments, the N-terminal end of the integrase of the POL polyprotein is fused to the C-terminal end of the aptamer-binding protein, optionally through a linker, and the N-terminal end of VPR is fused to the C-terminal end of the integrase of the POL polyprotein, optionally through a linker.

In some embodiments, the N-terminal end of the GAG polyprotein is fused to the C-terminal end of VPR, optionally through a linker, and the N-terminal end of the aptamer-binding protein is fused to the C-terminal end of the GAG polyprotein, optionally through a linker. In some embodiments, the N-terminal end of the GAG polyprotein is fused to the C-terminal end of the aptamer-binding protein, optionally through a linker, and the N-terminal end of VPR is fused to the C-terminal end of the GAG polyprotein, optionally through a linker.

In some embodiments, the N-terminal end of the GAG-POL polyprotein is fused to the C-terminal end of VPR, optionally through a linker, and the N-terminal end of the aptamer-binding protein is fused to the C-terminal end of the GAG-POL polyprotein, optionally through a linker. In some embodiments, the N-terminal end of the GAG-POL polyprotein is fused to the C-terminal end of the aptamer-binding protein, optionally through a linker, and the N-terminal end of VPR is fused to the C-terminal end of the GAG-POL polyprotein, optionally through a linker.

The present invention further relates to a population of lentiviral particles obtained by the method for producing lentiviral particles described above.

In some embodiments, the population of lentiviral particles comprises lentiviral particles comprising or consisting of:
(i) lentiviral proteins and/or genes or parts thereof, wherein the lentiviral integrase has impaired integration activity, as described hereinabove,
(ii) an RNA-guided nuclease or nickase, preferably a Cas9 protein, optionally fused to a mutated hyPB and/or VPR and/or P75, as described hereinabove, or a nucleic acid sequence coding therefor,
(iii) a guide RNA (gRNA) fused to an aptamer, preferably to at least one MS2 RNA tetraloop binding sequence, as described hereinabove, and
(iv) optionally, a fusion protein comprising (1) a first protein comprising or consisting of a GAG polyprotein, (2) a second protein comprising or consisting of an aptamer-binding protein, and optionally (3) a third protein comprising or consisting of a POL polyprotein, as described hereinabove, or a nucleic acid sequence coding therefor.

In some embodiments, the lentiviral particles in the population of lentiviral particles further comprise a transgene of interest, as described above.

In some embodiments, the lentiviral particles are suspended and stored in a suitable solvent, preferably a bio-compatible solvent (*e.g.,* biological buffer, physiological serum and the like).

In some embodiments, the lentiviral particles are stored at a temperature suitable for preventing nucleic acid degradation. In some embodiments, the lentiviral particles are stored at a temperature comprised from 4°C to -200°C. As used herein, the expression "from 4°C to -200°C" encompasses 4, 3, 2, 1, 0, -1, -2, -3, -4, -5, -6, -7, -8, -9, -10, -11, - 12, -13, -14, -15, -16, -17, -18, -19, -20, -30, -40, -50, -60, -70, -80, -90, -100, -120, -140, -160, -180, -200°C. In some embodiments, the lentiviral particles are stored at a temperature comprised between 4°C and -80°C. In some embodiments, the lentiviral particles are stored at a temperature comprised between 4°C and -20°C. In some embodiments, the lentiviral particles are stored at a temperature comprised between 4°C and 0°C. In some embodiments, the lentiviral particles are stored at a temperature comprised between 0°C and -200°C. In some embodiments, the lentiviral particles are stored at a temperature comprised between -20°C and -200°C. In some embodiments, the lentiviral particles are stored at a temperature comprised between -80°C and -200°C. In some embodiments, the lentiviral particles are stored at a temperature of -196°C.

In some embodiments, the lentiviral particles are substantially pure.

In some embodiments, the lentiviral particles further comprise at least one therapeutic agent.

In some embodiments, when the lentiviral particles comprise the fusion protein comprising a GAG polyprotein, an aptamer-binding protein, and optionally a POL polyprotein, this fusion protein (or the nucleic acid coding therefor) and the RNA-guided nuclease or nickase (or the nucleic acid coding therefor) may be comprised in distinct lentiviral particles.

In some embodiments, when the lentiviral particles comprise the fusion protein comprising a GAG polyprotein, an aptamer-binding protein, and optionally a POL polyprotein, this fusion protein (or the nucleic acid coding therefor) and the RNA-guided nuclease or nickase (or the nucleic acid coding therefor) are comprised in 2 distinct lentiviral particles.

In some embodiments, the population of lentiviral particles comprises:
a first lentiviral particle comprising or consisting of:
   (i) lentiviral proteins and/or genes or parts thereof, wherein the lentiviral integrase has impaired integration activity, as described hereinabove,
   (ii) an RNA-guided nuclease or nickase, preferably a Cas9 protein, optionally fused to a mutated hyPB and/or VPR and/or P75, as described hereinabove, or a nucleic acid sequence coding therefor, and
   (iii) a guide RNA (gRNA) fused to an aptamer, preferably to at least one MS2 RNA tetraloop binding sequence, as described hereinabove; and
a second lentiviral particle comprising or consisting of:
   (i) lentiviral proteins and/or genes or parts thereof, wherein the lentiviral integrase has impaired integration activity, as described hereinabove, and
   (ii) a fusion protein comprising (1) a first protein comprising or consisting of a GAG polyprotein, (2) a second protein comprising or consisting of an aptamer-binding protein, and optionally (3) a third protein comprising or consisting of a POL polyprotein, as described hereinabove, or a nucleic acid sequence coding therefor.

In some embodiments, the first lentiviral particle, the second lentiviral particle, or both, comprise the transgene of interest, as described hereinabove.

In some embodiments, the population of lentiviral particles comprises:
a first lentiviral particle comprising or consisting of:
   (i) lentiviral proteins and/or genes or parts thereof, wherein the lentiviral integrase has impaired integration activity, as described hereinabove, and
   (ii) an RNA-guided nuclease or nickase, preferably a Cas9 protein, optionally fused to a mutated hyPB and/or VPR and/or P75, as described hereinabove, or a nucleic acid sequence coding therefor; and
a second lentiviral particle comprising or consisting of:
   (i) lentiviral proteins and/or genes or parts thereof, wherein the lentiviral integrase has impaired integration activity, as described hereinabove,
   (ii) a fusion protein comprising (1) a first protein comprising or consisting of a GAG polyprotein, (2) a second protein comprising or consisting of an aptamer-binding protein, and optionally (3) a third protein comprising or consisting of a POL polyprotein, as described hereinabove, or a nucleic acid sequence coding therefor, and
   (iii) a guide RNA (gRNA) fused to an aptamer, preferably to at least one MS2 RNA tetraloop binding sequence, as described hereinabove.

In some embodiments, the first lentiviral particle, the second lentiviral particle, or both, comprise the transgene of interest, as described hereinabove.

In some embodiments, when the lentiviral particles comprise the fusion protein comprising a GAG polyprotein, an aptamer-binding protein, and optionally a POL polyprotein, this fusion protein (or the nucleic acid coding therefor) and the RNA-guided nuclease or nickase (or the nucleic acid coding therefor) are comprised in 3 distinct lentiviral particles.

In some embodiments, the population of lentiviral particles comprises:
a first lentiviral particle comprising or consisting of:
   (i) lentiviral proteins and/or genes or parts thereof, wherein the lentiviral integrase has impaired integration activity, as described hereinabove, and
   (ii) an RNA-guided nuclease or nickase, preferably a Cas9 protein, optionally fused to a mutated hyPB and/or VPR and/or P75, as described hereinabove, or a nucleic acid sequence coding therefor; and
a second lentiviral particle comprising or consisting of:
   (i) lentiviral proteins and/or genes or parts thereof, wherein the lentiviral integrase has impaired integration activity, as described hereinabove, and
   (ii) a fusion protein comprising (1) a first protein comprising or consisting of a GAG polyprotein, (2) a second protein comprising or consisting of an aptamer-binding protein, and optionally (3) a third protein comprising or consisting of a POL polyprotein, as described hereinabove, or a nucleic acid sequence coding therefor; and
a third lentiviral particle comprising or consisting of:
   (i) lentiviral proteins and/or genes or parts thereof, wherein the lentiviral integrase has impaired integration activity, as described hereinabove, and
   (ii) a guide RNA (gRNA) fused to an aptamer, preferably to at least one MS2 RNA tetraloop binding sequence, as described hereinabove.

In some embodiments, the first lentiviral particle, the second lentiviral particle, the third lentiviral particle, or two or all three of them, comprise the transgene of interest, as described hereinabove.

In some embodiments, when the lentiviral particles comprise the fusion protein comprising a GAG polyprotein, an aptamer-binding protein, and optionally a POL polyprotein, this fusion protein (or the nucleic acid coding therefor) and the RNA-guided nuclease or nickase (or the nucleic acid coding therefor) are comprised in 4 distinct lentiviral particles.

In some embodiments, the population of lentiviral particles comprises:
a first lentiviral particle comprising or consisting of:
   (i) lentiviral proteins and/or genes or parts thereof, wherein the lentiviral integrase has impaired integration activity, as described hereinabove, and
   (ii) an RNA-guided nuclease or nickase, preferably a Cas9 protein, optionally fused to a mutated hyPB and/or VPR and/or P75, as described hereinabove, or a nucleic acid sequence coding therefor; and
a second lentiviral particle comprising or consisting of:
   (i) lentiviral proteins and/or genes or parts thereof, wherein the lentiviral integrase has impaired integration activity, as described hereinabove, and
   (ii) a fusion protein comprising (1) a first protein comprising or consisting of a GAG polyprotein, (2) a second protein comprising or consisting of an aptamer-binding protein, and optionally (3) a third protein comprising or consisting of a POL polyprotein, as described hereinabove, or a nucleic acid sequence coding therefor; and
a third lentiviral particle comprising or consisting of:
   (i) lentiviral proteins and/or genes or parts thereof, wherein the lentiviral integrase has impaired integration activity, as described hereinabove, and
   (ii) a guide RNA (gRNA) fused to an aptamer, preferably to at least one MS2 RNA tetraloop binding sequence, as described hereinabove; and
a fourth lentiviral particle comprising or consisting of:
   (i) lentiviral proteins and/or genes or parts thereof, wherein the lentiviral integrase has impaired integration activity, as described hereinabove, and
   (ii) a transgene of interest, as described hereinabove.

The present invention further relates to a method for producing lentiviral particles, comprising the steps of:
(i) transfecting lentiviral producer cells cultured in a suitable culture medium with the composition according to the invention, and
(ii) collecting lentiviral particles in the culture medium of said lentiviral producer cells.

In some embodiments, lentiviral producer cells are transfected with 1, 2, 3, 4 or the 5 nucleic acids of the composition described above. For instance, it is conceivable that different batches of lentiviral producer cells be transfected with different nucleic acids of the composition described above, so as to produce different lentiviral particles.

Cells may be transfected by methods known in the art; non-limitative examples include lipofection, electroporation, sonoporation, gene gun, microinjection or chemical-based such as calcium phosphate.

In some embodiments, the cells are transfected simultaneously and/or separately with the nucleic acid molecules of the composition. In some embodiments, the cells are transfected simultaneously with the nucleic acid molecules of the composition. In some embodiments, the cells are transfected separately with the nucleic acid molecules of the composition.

In some embodiments, the lentiviral particles are obtainable by a split system, *e.g.,* a transcomplementation system (vector/packaging system), by transfecting *in vitro* a permissive cell (*e.g.,* HEK 293T cells) with a plasmid containing the components of the lentiviral vector genome comprising the gag, pol and *env* sequences encoding the polyproteins GAG, POL and the envelope polypeptides, or a portion of these polypeptides sufficient to enable formation of retroviral particles.

Suitable cells for the production of the lentiviral particles include, but not limited to, eukaryotic and prokaryotic cells and/or cell lines. Non-limiting examples of such cells or cell lines generated from such cells include, *e.g.,* COS, CHO *(e.g.,* CHO-S, CHO-K1, CHO-DG44, CHO-DUXB11, CHO-DUKX, CHOK1SV), VERO, MDCK, WI38, V79, B14AF28-G3, BHK, HaK, NS0, SP2/0-Ag14, HeLa, HEK293 (e.g., HEK293-F, HEK293-H, HEK293-T), and perC6 cells as well as insect cells such as *Spodoptera fugiperda* (Sf), or fungal cells such as *Saccharomyces, Pichia* and *Schizosaccharomyces.*

In some embodiments, the lentiviral particles are secreted by the lentiviral producer cells into their culture medium. In some embodiments, the lentiviral particles are collected in the culture medium of the lentiviral producer cells.

In some embodiments, the lentiviral particles are collected from 12 hours to one week after transfection, preferably from 24 to 72 hours after transfection.

In some embodiments, the lentiviral particles are purified from the culture medium of the lentiviral producer cells. Purification of lentiviral particles to enhance the concentration can be accomplished by any suitable method, such as by density gradient purification *(e.g.,* cesium chloride (CsCl)), by chromatography techniques *(e.g.,* column or batch chromatography), or by ultracentrifugation. For example, the lentiviral particle of the invention can be subjected to two or three CsCl density gradient purification steps. The lentiviral particle is desirably purified from infected cells using a method that comprises lysing cells, applying the lysate to a chromatography resin, eluting the lentiviral particle from the chromatography resin, and collecting a fraction containing the lentiviral particle.

The present invention further relates to a producer cell, comprising (*i.e.,* transfected with) the composition according to the invention.

In some embodiments, the producer cell comprising the composition according to the invention produces lentiviral particles. Typically, the lentiviral particles are budding from the plasma membrane of the cell.

In some embodiments, the producer cell is from an immortalized cell line. In some embodiments, the producer cell is from a primary cell culture.

In some embodiments, the producer cell is a eukaryotic cell. In some embodiments, the producer cell is an animal cell or a plant cell. In some embodiments, the producer cell is an animal cell. In some embodiments, the producer cell is a vertebrate cell or an insect cell. In some embodiments, the producer cell is a vertebrate cell. In some embodiment, the producer cell is a mammalian cell.

In some embodiments, the producer cell is a non-human mammal cell *(e.g.,* non-human primate, mouse, rat). In some embodiments, the producer cell is a human cell. Cultivated cell lines well known in the art *(e.g.,* HeLa cells, HEK293 cells, HAP-1 cells and the like). In some embodiments, the producer cell is from a HEK293 cell line. In some embodiments, the producer cell is from a HEK293T cell line.

In some embodiments, the producer cell comprises one or more mutations compared to the cell line it is derived from. In some embodiments, the producer cell is unmodified compared to the cell line it is derived from.

Methods for maintaining cells in culture are well known in the art. In some embodiments, the producer cells are maintained in a suitable culture medium, *e.g*., Dulbecco's modified Eagle medium (DMEM). In some embodiments, the producer cells are typically cultivated at 37°C, under 95% air and 5% CO₂.

The present invention further relates to a method for site-specific integration of a transgene of interest into the genome of a target cell, comprising infecting said cell with the population of lentiviral particles described above.

In some embodiments, the method is an *in vitro* method.

In some embodiments, the lentiviral particles comprise the transgene of interest. In some embodiments, some lentiviral particles in the population of lentiviral particles comprises the transgene of interest.

In some embodiments, the transgene of interest is comprised in one or several or all of the lentiviral particles of the population of lentiviral particles. In some embodiments, the lentiviral particles do not contain the transgene of interest, which is delivered to the target cells by a suitable method, such as by transfection (e.g., lipofection, electroporation and the like). In some embodiments, the transgene of interest is delivered to the target cells before the lentiviral particles. In some embodiments, the transgene of interest is delivered to the target cells concomitantly with the lentiviral particles. In some embodiments, the transgene of interest is delivered to the target cells after the lentiviral particles.

In some embodiments, the method aims at editing the genome of the target cell, in particular to perform targeted insertion of a transgene of interest.

In some embodiments, the target cells can be infected with the lentiviral particles of the invention. In some embodiments, the target cells express at their extracellular surface at least one protein, or part thereof, or protein complex, that can be recognized by the lentiviral particles, in particular by a surface protein of the lentiviral particles. In some embodiments, this at least one protein or protein complex allows the binding (or anchorage) and/or the entry of the lentiviral particles inside the target cell.

In some embodiments, the target cells express the CD4, CCR5 and/or CXCR4 surface proteins. Typically, CD4, CCR5 and/or CXCR4 interact with HIV-1 surface proteins Gp120 and/or Gp41.

In some embodiments, the target cells are primary cells or cells from a cell line suitable for lentiviral infection, e.g., HEK 293T cells, CHO cells, HSC cells, iPS cells, primary immune cells, primary T-cells, primary NK cells, primary macrophages, primary hepatocytes, K-562 cells and Jurkat-T cells.

In some embodiments, the target cell artificially expresses at least one surface protein that allows lentiviral particles' binding and/or entry. By "artificially express", it is meant that the cell does not express the surface protein endogenously, but has been modified or engineered to express the surface protein, either transiently or stably.

In some embodiments, the target cells are cells isolated from an organism, preferably a mammalian organism, more preferably a human organism. In some embodiments, the target cells are myeloid cells. In some embodiments, the target cells are lymphocytes.

In some embodiments, the method is used to perform targeted gene insertion in a population of target cells extracted from an organism, maintained *in vitro,* and reintroduced into the organism. According to this embodiment, the method is said to be *ex vivo.*

It is to be understood that, upon delivery of the content of the lentiviral particles into the target cells, the RNA-guided nuclease or nickase (*e.*g., Cas9) forming a complex with the gRNA comprising at least one aptamer (*e.*g., MS2 RNA tetraloop binding sequence) will interact with the aptamer-binding protein (*e.g.,* MCP) fused to the integrase (from the lentiviral vector with impaired integration activity and/or from the fusion protein comprising a third protein comprising or consisting of a POL polyprotein, when present, as described above). In particular, this interaction occurs through aptamer/aptamer-binding protein interaction (*e.g.,* MS2/MCP).

In some embodiments, the site specificity of the gRNA directs the RNA-guided nuclease or nickase/integrase complex activity towards a specific region or site of the genome of the target cell *(e.g.,* to a safe harbor locus or within or in close proximity to a gene in the target cell's genome).

In some embodiments, the transgene of interest is inserted at the site specifically recognized by the gRNA. In some embodiments, the transgene of interest is inserted at a distance from 1 to 20 nucleotides from the sequence specifically recognized by the gRNA, wherein "from 1 to 20" encompasses 1, 2, 3, 4, 5, 6, 7, 8, 9, 10, 11, 12, 13, 14, 15, 16, 17, 18, 19 and 20.

In some embodiments, the mutated integrase activity results in targeted gene integration, also referred to as site-specific integration, of the transgene of interest.

In some embodiments, the transgene of interest is not inserted into a coding sequence and/or a regulatory sequence of the genome of the target cell. In some embodiments, the targeted gene insertion resulting from the use of the method of the invention does not disrupt, alter nor decreases the expression of one or more genes.

In some embodiments, the transgene of interest is inserted into a coding sequence and/or a regulatory sequence of the genome of the target cell, resulting in the partial or complete inactivation of the coding sequence.

In some embodiments, the transgene of interest is inserted by non-homologous end-joining (NHEJ). In some embodiments, the transgene of interest is inserted by homologous recombination (HR).

In some embodiments, the inserted transgene of interest is expressed by the cell. In some embodiments, the inserted transgene of interest is stably expressed by the cell. In some embodiments, the expression of the inserted transgene of interest is under the control of a promoter or a regulatory element.

In some embodiments, site-specific integration of the transgene of interest is controlled by sequencing the region of the genome surrounding the target site, *i.e.,* the site recognized the gRNA.

In some embodiments, the ratio of the number of lentiviral particles to the number of cells (multiplicity of infection, MOI) is MOI 0.5 to MOI 40.

The present invention further relates to the population of lentiviral particles as described above (plus optionally, the transgene of interest when said transgene of interest is not comprise in a lentiviral particle of the population of lentiviral particles), for use as a drug.

The present invention further relates to the population of lentiviral particles as described above (plus optionally, the transgene of interest when said transgene of interest is not comprise in a lentiviral particle of the population of lentiviral particles), for use for site-specific integration of a transgene of interest into the genome of a target cell.

The present invention further relates to the population of lentiviral particles as described above (plus optionally, the transgene of interest when said transgene of interest is not comprise in a lentiviral particle of the population of lentiviral particles), for use in the treatment of a disease in a subject in need thereof. It also relates to a method for treating a disease in a subject in need thereof, comprising administering to the subject a therapeutically effective amount of the population of lentiviral particles as described above (plus optionally, the transgene of interest when said transgene of interest is not comprise in a lentiviral particle of the population of lentiviral particles).

In some embodiments, treatment of the disease occurs by site-specific integration of the transgene of interest into the genome of a cell of the subject in need thereof.

In some embodiments, the disease is a genetic disease. In some embodiments, the genetic disease is characterized in that at least one gene is partially or completely silenced, inactivated or malfunctional. In some embodiments, the genetic disease is characterized in that at least one gene in a cell of the subject in need thereof encodes at least one non-functional protein. In some embodiments, the genetic disease is characterized in that at least one gene in a cell of the subject in need thereof encodes at least one protein or polypeptide having deleterious effects in the cell.

In some embodiments, the disease is cancer. In some embodiments, the cancer is blood cancer (e.g., leukemia).

Typically, the targeted gene insertion restores a normal expression and function of the at least one gene involved in the disease.

In some embodiments, the population of lentiviral particles is to be administered, either before, concomitantly with or after, a second therapeutic agent.

### BRIEF DESCRIPTION OF THE DRAWINGS

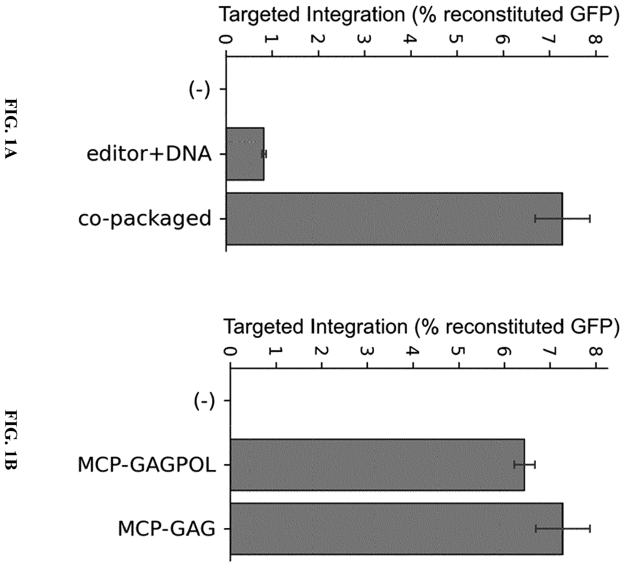

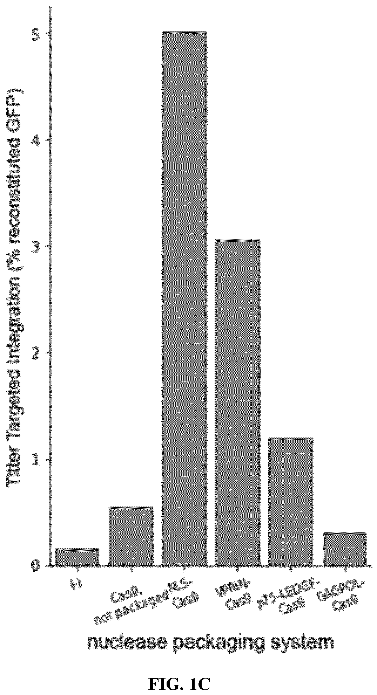

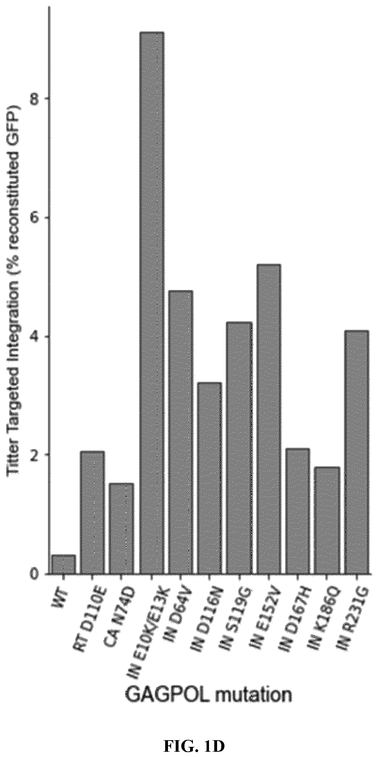

**Figures 1A-D** relate to several packaging strategies for achieving programmable insertion. **Fig. 1A** shows that simultaneous loading with both Nuclease and payload DNA (co-packaged) are more efficient in targeted integration than separate addition of the components (editor + DNA). **Fig. 1B** shows the targeted integration of LV payload with the aptamer binding protein (MCP) fused to either the polyprotein GAG or the polyproten GAG-POL. **Fig. 1C** shows the targeted integration of LV payload with the different packaging strategies: co-packaging with either NLS (NLS-Cas9), integrase fused with viral protein R (VPRIN-Cas9), LEDGF (p75-LEDGF-Cas9) and Gag-Pol (GAGPOL-Cas9). **Fig. ID** shows how integrase mutants lentiviral proteins determine efficiencies of on-target integration levels upon simultaneous SpCas9 nuclease RNP delivery and LV vector infection.

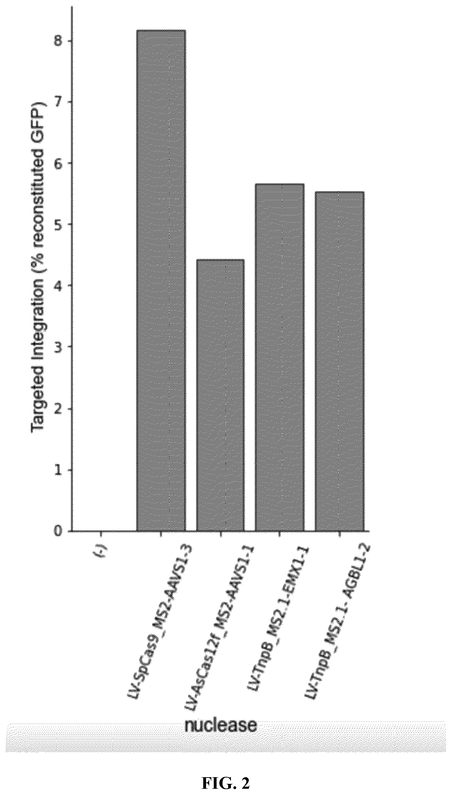

**Figure 2** is a graph showing targeted DNA integration with different RNA guided nucleases: SpCas9, SaCas9, Cas12f and TnpB can be efficiently used for targeted delivery.

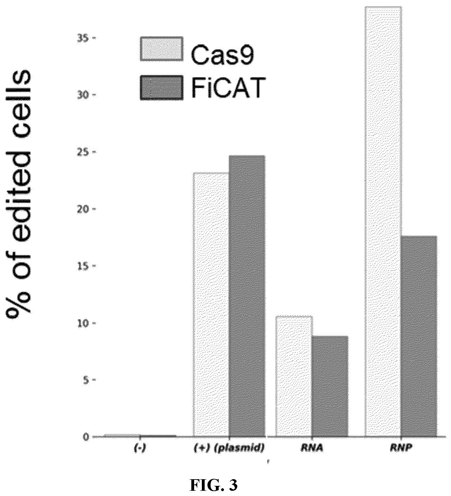

**Figure 3** shows the percentage of NHEJ activated cells following the delivery of either Cas9 nuclease or Cas9 fused to a Programmable Transposase (FiCAT), in a lentiviral particle using the MS2 system, in either DNA, RNA or protein form. Loading is measured by NHEJ performed in a reporter cell line.

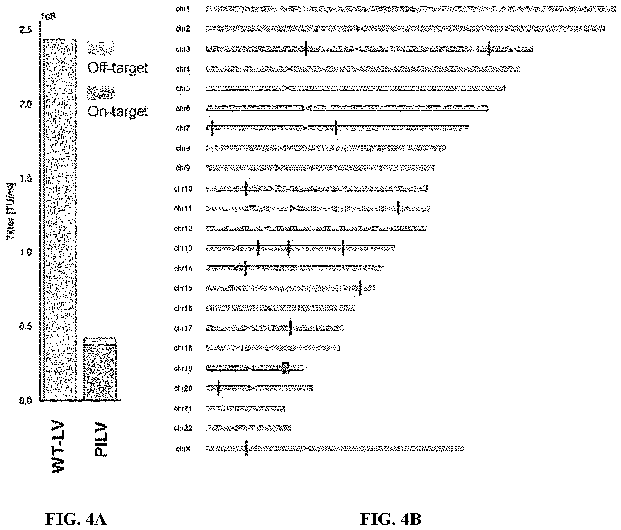

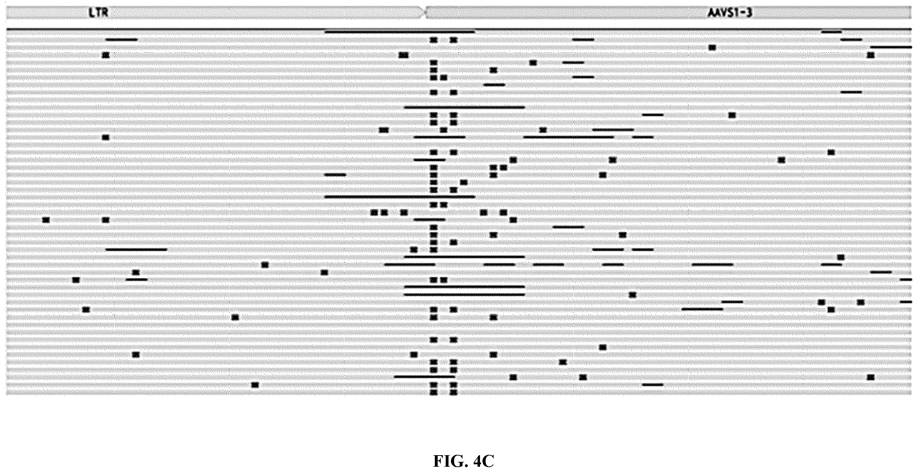

**Figures 4A-C** relate to the integrated junction sequencing. **Fig. 4A** shows a comparison of the titer of targeted integration (TU/mL) obtained with a wild type lentiviral vector (WT-LV) and the co-delivery of a mutated integrase fused to MCP, a Cas9 protein and a MS2-containg gRNA (PILV), in HEK293T cells, with a comparison between on-target and off-target integration. **Fig. 4B** shows insertion events detected on each chromosome of HEK293T cells; the larger band on chromosome 19 corresponds to on-target integration. **Fig. 4C** exemplifies the integrated junction sequencing.

### EXAMPLES

The present invention is further illustrated by the following example.

### Example 1: targeted DNA integration with lentiviral vectors

### Materials and methods

### Plasmid construction

The following plasmids were obtained from Addgene:
- hCas9 (plasmid #41815), a pcDNA^{™}3.3-TOPO^{™} plasmid expressing human codon-optimized Cas9 nuclease under the control of a CMV promoter;
- psPAX2 (plasmid #12260), an empty second-generation lentiviral packaging plasmid comprising the *gag*, *pol*, *tat* and *rev* genes;
- pCMV-VSV-G (plasmid #8454), a plasmid expressing the G glycoprotein of the vesicular stomatitis virus under the control of a CMV promoter;
- pMDLg/pRRE (plasmid #12251), a third-generation lentiviral packaging plasmid expressing HIV-1 Gag (coding for the virion main structural proteins), HIV-1 Pol (coding for HIV-1 protease, HIV-1 reverse transcriptase and HIV-1 integrase) and HIV-1 Rev response element (RRE, binding site for the Rev protein which facilitates export of the RNA from the nucleus);
- pRSV-Rev (plasmid #12253), a third-generation lentiviral packaging plasmid expressing the Rev protein.

Mutations in the integrase were obtained with QuikChange Lightning Multi Site-Directed Mutagenesis Kit (Agilent # 210513). The sequences of integrase mutants are disclosed in SEQ ID NO: 2 to SEQ ID NO: 9.

All the remaining vectors were built by Golden Gate assembly using Esp3I and T4 ligase. Plasmid pSICO (Addgene reference Plasmid #41815; a generic vector comprising a transgene of interest) of SEQ ID NO: 74, pRRL dual of SEQ ID NO: 75 (a generic vector comprising a transgene of interest), psMCP-GAG of SEQ ID NO: 76, and psMCP-GAGPOL of SEQ ID NO: 77 were used.

### Cell culture

HEK293T cells (ATCC CRL-3216) were grown in Dulbecco's modified Eagle medium (DMEM), supplemented with high glucose (Gibco, Thermo Fisher), 10 % fetal bovine serum, 2 mM glutamine and 100 U penicillin, 0.1 mg/mL streptomycin.

K-562 cells (ATCC CRL-3343) and Jurkat-T cells (Clone E6-1, ATCC IB-152) were grown in RPMI 1640 medium (Gibco) supplemented with 10 % FBS, 1 % penicillin-streptomycin (Gibco) and 1 % GlutaMAX (100x) (Gibco).

All cell lines were grown at 37°C under 95 % air and 5 % CO₂.

### Lentiviral production

Lentiviral vectors were produced following protocol available at Addgene website at https://www.addgene.org/protocols/lentivirus-production/, and reproduced below:

### Introduction

*This protocol can be used to produce lentivirus from a lentiviral vector transfected into Lenti-X 293T cells using a polyethyenimine (PEI) transfection protocol. This procedure can be modified for alternative packaging cell lines or transfection reagents. Once produced, lentivirus can be used for a variety of downstream applications such as stable-cell line generation.*

### Workflow timeline

*Day 0: Seed 293Tpackaging cells*
*Day 1 (pm): Transfect packaging cells*
*Day 2 (am): 18 hours post transfection. Remove media, replace with fresh media*
*Day 3-4 (am): Harvest virus*

### Reagent Preparation

*1. DMEM Complete: 10% v*/*v FBS and 4 mM L-alanyl-L-glutamine*
*To a 500 mL bottle of DMEM high glucose, add 55 mL of heat inactivated FBS and 11 mL of 200 mM L-alanyl-L-glutamine. Store at 4°C.*
*2. 25 mM chloroquine diphosphate*
*Dissolve 0.129 g of chloroquine diphosphate salt in 10 mL of sterile water.*
*Filter sterilize through a 0.22 µm filter.*
*Aliquot 50-100 µL and store at -20°C.*
*Aliquots can be thawed and stored at 4°C prior to use. Thawed aliquots should be discarded after 1-2 months.*
*3. 1 mg*/*mL PEI, linear MW 25,000 Da*
*Dissolve 100 mg of powder in 100 mL of deionized water.*
*While stirring, slowly add hydrochloric acid until the solution clears.*
*Check the pH of the solution.*
*Use hydrochloric acid or sodium hydroxide to adjust the pH to 7.0. Typically the solution will be basic and will need adjustment with hydrochloric acid first.*
*Allow the solution to mix for 10 min and then recheck the pH to ensure that it has not drifted.*
*Filter the solution through a 0.22 µm membrane.*
*Aliquot 500-1000 µL into sterile tubes.*
*Store the tubes at -80°C.*
*After thawing, the solution can be stored at 4°C for up to 2 months. After 2 months, discard the tube and thaw a new working stock.*
*The optimal mass DNA:mass PEI ratio will need to be empirically determined for each new batch of 1 mg*/*mL PEI and for each cell line.*

### Procedure

*Seed 293 Tpackaging cells at 3.8*× *10⁶ cells per plate in DMEM complete in 10 cm tissue culture plates.*
*Incubate the cells at 37°C, 5% CO2 for ~20 hours.*
*Gently aspirate media, add 10 mL fresh DMEM complete containing 25 µM chloroquine diphosphate and incubate ~5 hours.*
*For 10 mL of DMEM complete, add 10 µL of 25 mM chloroquine diphosphate. Prepare a mixture of the 3 transfection plasmids:*
   *psPAX2: 1.3 pmol*
   *pMD2. G: 0.72 pmol*
   *Transfer plasmid: 1.64 pmol*
   *OptiPro SFM to total volume of 500 µL*
*Dilute the above 500 µL mixture into 500 µL PEI-OptiPro SFM with enough PEI such that the ratio of µg DNA:µg PEI is 1:3 (1000 µL total per 10 cm dish).*
*Using transfer plasmid pHAGE TRE dCas9-KRAB (total ug of plasmid DNA 27.8 µg), this would be 83.4 µL of 1 mg*/*mL PEI in 416.6 µL of OptiPro SFMper 10 cm dish.*
*Gently add the diluted PEI to the diluted DNA. Add the diluted PEI dropwise while gently flicking the diluted DNA tube. Incubate the mixture 15-20 min at room temperature.*
*Carefully transfer the transfection mix to the Lenti-X 293Tpackaging cells. Add the transfection mix dropwise being careful not to dislodge the cells. Incubate the cells for 18 hours, or until the following morning.*
*The following morning, carefully aspirate the media. Replace the media with 15 mL of DMEM complete.*
*Incubate the cells.*
*Virus can be harvested at 48, 72, and 96 hours post transfection in individual harvests or a combined harvest where all the individual harvests are pooled. If pooling harvests, transfer the harvested media to a polypropylene storage tube and store at 4°C between harvest.*
*Centrifuge the viral supernatant at ~500 g for 5 minutes to pellet any packaging cells that were collected during harvesting.*
*Filter supernatant through a 0. 45 µm PESfilter.*
*The viral supernatant can be stored at 4°C for several hours but should be aliquotted and snap frozen in liquid nitrogen and stored at -80°C as soon as possible to avoid loss of titer.*

Cells were produced in 10-cm dishes seeded with 4.9 × 10⁶ HEK293T cells a day prior to transfection, using 0.72 pmol of pCMV-VSV-G envelope plasmid, 1.64 pmol of pSICO or pRRL dual payloads (transfer plasmid) and 1.30 pmol psPAX2 plasmid, either in wild-type form or with pol mutants.

For nuclease packaging, 0.65 pmol of psPAX2 and 0.65 pmol of psMCP-GAG or psMCP-GAGPOL plasmids were used together with 0.65 pmol of the nuclease plasmid.

For transfection, plasmids were mixed in 500 µL of Optimem and 100 mg of polyethyenimine (PEI). Two days after plasmid transfection, the supernatant was harvested and filtered and centrifuged over night at 4000 g and 4°C. Supernatant was discarded and lentiviral particles were resuspended to achieve 100x vector concentration.

### Determination of nuclease activity

For VPR/IN/LEDGF nuclease fusion activity, traffic light reporter (TLR) (Certo et al., 2011. Nat Methods. 8(8):671-676) HEK293T cell line, which contains an out-of-frame mCherry ORF that gets reconstituted by NHEJ activity upon Cas9 targeting, was used. Fluorescence was measured by flow cytometry using a BD LSR Fortessa instrument (Yellow green 561 nm laser with 610/20 filter).

SaCas9, AsCas12f and TnpB editing and MS2 scaffold in gRNA impact HEK293T were assessed by transfecting cells with gRNA in p12 cells with 240 000 cells/well. Three days after nuclease addition, cells were pelleted and DNA extracted with KAPPA quick extract. PCR amplification of the respective target site was performed, and sequenced on Illumina sequencing by synthesis MiSeq^{™} platform with MiSeq Reagent Kit v2 (300 cycles, 2× 150 configuration).

### Determination of on-target integration

A reporter cell line (Hershey) was built to asses targeted integration, in which reconstitution of a fluorescent protein ORF upon on-target integration led to a measurable fluorescent signal (GFP).

A viral payload plasmid containing ½ of Emerald GFP (emGFP) and ½ of intron was packaged in a lentiviral vector with corresponding packaging system and/or integrase mutant. A promoter-less C-terminal (C-t) half of emGFP preceded by a splicing acceptor was randomly inserted in the genome of HEK293T cells to build a reporter cell line. A "target site" was added upstream of the C-t emGFP.

To perform the experiment, 200 000 HEK293T Hershey reporter cells were seeded in 12 well/plates and infected with lentiviral particles the next day, at the same time of transfection, that led to emGFP reconstitution upon addition of a payload plasmid.

Lentiviruses were produced using standard protocols. Four days after infection, emGFP fluorescent signal was measured by FACS (BD LSR Fortessa instrument; blue 488 nm laser with 530/30 filter, Yellow Green 561 nm laser with 610/20 filter).

### Integrated junction sequencing

INSERTseq method combines targeted amplification of integrated DNA, UMI-based correction of PCR bias and Oxford Nanopore long-read sequencing for robust analysis of DNA integration in a genome. INSERT-seq is capable of detecting events occurring at a frequency of up to 0.1 %. INSERT-seq presents a complete handling of all insertions independently of repeat size.

### Library preparation and sequencing

DNA was extracted using Nanobind kit (Circulomics, catalog no. NB-900-001-01), sheared to ~ 2 kb fragments using g-TUBE^{™} (Covaris, catalog no. 520079). WGP primer mix from Nanopore PCR Barcoding Kit (SQK-PBK004) was additionally added to the second PCR. Sequencing was performed in Flongle R9.4.1 flow cells obtaining a total output of ~ 300 000 reads. For the calculation of the limit of detection (LOD), a monoclonal sample from HEK293T cells with one true lentiviral insertion was diluted with WT genomic material at the proportions 1/100, 1/1 000 and 1/10 000. Dilutions were sequenced a Flongle R9.4.1 flowcell obtaining a sequencing output of 500 000, 500 000 and 150 000 reads respectively. The analysis was performed following the INSERTseq analysis.

### Integration site analysis

Nanopore raw reads were base-called using Guppy 4.0.11 (made available by ONT via their webpage https://community.nanoporetech.com).

Read quality was assessed with NanoStats NanoQC and NanoPlot from NanoPack (De Coster et al., 2018. Bioinformatics. 34(15):2666-2669). Reads were filtered by quality (> 10) and length (> 200) with NanoFilt from NanoPack. Reads were clustered by UMI using a combination and adaptation of two previously published pipelines (pipeline-umi-amplicon distributed by ONT https://github.com/nanoporetech/pipeline-umi-amplicon). The clustering was performed by extracting the UMI sequences with Python scripting, sequences were clustered with vsearch (Rognes et al., 2016. PeerJ. 4:e2584) and the consensus sequence of the clusters was obtained by performing two rounds of polishing with racon (Vaser et al., 2017. Genome Res. 27(5):737-746) and two rounds of medaka (https://github.com/nanoporetech/medaka).

For the analysis of insertions, reads were filtered to force the presence of used adapters and trimmed to remove the adapter and insert sequence from the read with cutadapt (Martin, 2011. EMBnet j. 17(1):10-12). Reads were mapped against the reference genome with minimap2 (Li, 2018. Bioinformatics. 34(18):3094-3100) "map-ont" default parameters and filtered with Python scripting, selecting uniquely mapping reads with a map quality higher than 30. A first output is returned with bedtools (Quinlan & Hall, 2010. Bioinformatics. 26(6):841-842) in bed format containing all mapped reads. Afterwards, a peak calling step is performed with Python scripting where peaks are filtered by shape. A peak is considered to pass the shape filter when the Residuals Sum of Squares (RSS) of fitting the peak coverage to a beta distribution is lower than 1.

### Implementation of the optimized INSERTseq protocol

Genomic DNA was extracted, fragmented, end-repaired and A-tailed followed by ligation of an adaptor that contains an UMI for read clustering and a barcode for sample demultiplexing in the computational pipeline. Reads were clustered by UMI, integration sequence and adapters were filtered and trimmed, reads were mapped against the reference genome and significant peaks were reported and annotated.

### Results

### Nuclease packaging strategies

Given that lentiviral DNA integrates in double-strand breaks (DSB), it was reasoned that packaging functional Cas9-gRNA into a lentiviral particle should generate targeted integration.

Six different packaging system configurations were tested to achieve targeted integration via packaging of SpCas9 protein in lentiviruses:
(i) co-expression of SpCas9 upon vector production,
(ii) co-expression of SpCas9 fusion with virion-targeted protein Vpr p6-GAG interacting domain, as previously described (Indikova & Indik, 2020. Nucleic Acids Res. 48(14):8178-8187),
(iii) SpCas9 fusion with the integrase protein,
(iv) fusion to VPR integrase as previously described (Montagna et al., 2018. Mol Ther Nucleic Acids. 12:453-462),
(v) fusion to the integrase-binding domain of the LEDGF-p75 chromatin docking factor as previously described (Hare et al., 2009. PLoSPathog. 5(1):e1000259), and
(vi) direct fusion to GAGPOL polyprotein in the packaging plasmid.

SpCas9 protein was detected in all of the packaging systems, but no non-homologous end joining (NHEJ) editing was detected upon lentiviral administration to cultured cells. One hypothesis was that lack of editing could be due to missing SpCas9 gRNA, as U6-driven expression localizes the gRNA in the nucleus, and cytoplasmic gRNA expression has been shown to rescue packaging of gRNA in lentivirus-derived vesicles.

An MCP-GAGPOL packaging fusion and MS2 stem-loop containing SpCas9 gRNA were used to recruit the gRNA to lentiviral particles. Using this configuration, the editing activity upon transduction with lentiviral particles was rescued **(****Fig. 1A****).**

The efficiency of targeted integration was compared between lentiviral particles comprising the aptamer binding protein MS2 coat protein (MCP) fused either the lentiviral polyprotein GAG-POL or the polyprotein GAG alone **(****Fig. 1B****).** Both conditions led to a similar targeted integration rate.

Targeted integration was observed upon simultaneous lentiviral infection and Cas9 RNP delivery in Jurkat-T cells. Four different packaging strategies were used to generate a programmable lentivirus (PILV): Cas9 was co-packaged with either NLS (NLS-Cas9), with the integrase fused with viral protein R (VPRIN-Cas9), with LEDGF (p75-LEDGF-Cas9) or with Gag-Pol (GAGPOL-Cas9). NLS-Cas9 and VPRIN-Cas9 produced the highest insertion rate **(****Fig. 1C****).**

Several integrase (IN) mutants were tested, and all showed better targeted integration performances than the wild-type integrase **(Fig. ID).**

Finally, the proportion of NHEJ-activated cells was compared when the DNA payload was delivered using an editing tool that was either a Cas9 nuclease, or a fusion protein comprising a Cas9 and a programmable transposase (PT condition), in a lentiviral particle using the MS2 system. In addition, the editing tool was provided in either DNA, RNA or protein (RNP) form.

### Compatibility with other nucleases

Given the vast repertoire of programable nucleases that have been described since SpCas9, the programmable lentivirus packaging system (PILV) was tested with different programable nucleases (AsCas12 and TnpB). Targeted integration was detected in all of the systems **(****Fig. 2****),** showing that the system is compatible with any nuclease enzyme.

### Co-delivery with a programmable transposase

Using the MCP-MS2 system, either Cas9 or a fusion protein comprising Cas9 and a hyperactive PiggyBac transposase (FiCAT, with R372A/K375A/D450N substitutions) were loaded in lentiviral particles. Editing activity was measured in traffic-light reporter HEK293T cells transduced with lentiviral particles loaded with the gene editors either in mRNA or protein (RNP) form **(****Fig. 3****).**

The results show gene editing activity for both conditions, although better results were obtained when the gene editor was delivered in protein form.

### Measurement of on-target integration

On-target integration was measured using the INSERTseq approach as illustrated in **Fig. 4B** and **Fig. 4C****.**

As shown on **Fig. 4A****,** the overall integration and the on-target integration activity were compared between a wild-type lentiviral particle (WT-LV) and the programmable lentivirus (PILV). PILV enabled precise on-target integration compared to WT-LV, making it an optimal system for therapeutic applications.

### Conclusion

Targeted DNA integration of gene sized fragments in mammalian genomes can enable precise addition of therapeutic messages and new functionalities. Lentiviral vectors are therapeutically used for uncontrolled insertion of transgenes both for *ex vivo* applications for advance cell therapy and *in vivo* for gene therapy. By mutating the integrase protein and incorporating SpCas9 nuclease in lentiviral particles we observe that lentiviral vectors can be reprogramed to precisely integrate transgenic DNA.

Precise DNA integration was consistent in immortalized cell models and primary cells. PILV could also deliver a transgene in the ROSA26 locus in a mouse liver. We also detected PILV cis integration upon simultaneous delivery of two transgenes, providing a robust method for multiple KO-KI in *ex vivo* models. By changing two packaging vectors upon lentiviral production, lentiviral vectors can be made programable enabling a targeted DNA addition with applications in *ex vivo* cell manufacturing, *in vivo*

## Claims

1. A composition comprising:
(i) a nucleic acid encoding a lentiviral vector with impaired integration activity, optionally comprising an aptamer-binding protein,
(ii) a nucleic acid encoding a polypeptide or protein comprising an RNA-guided nuclease or nickase,
(iii) a nucleic acid encoding a guide RNA (gRNA) fused to an aptamer,
(iv) a nucleic acid encoding a transgene of interest, and
(v) optionally, a nucleic acid encoding a fusion protein comprising:
- a first protein comprising or consisting of a GAG polyprotein,
- a second protein comprising or consisting of an aptamer-binding protein, and
- optionally, a third protein comprising or consisting of a POL polyprotein.

2. The composition according to claim 1, wherein:
- said aptamer-binding protein is a MS2 bacteriophage coat protein (MCP), preferably sharing at least 75 % identity with SEQ ID NO: 61, and
- said aptamer is a MS2 RNA tetraloop binding sequence, preferably sharing at least 75 % identity with SEQ ID NO: 63.

3. The composition according to claim **1** or **2,** wherein said RNA-guided nuclease or nickase is not fused to an integrase.

4. The composition according to any one of claims **1** to **3,** wherein said RNA-guided nuclease or nickase is a Cas protein.

5. The composition according to any one of claims **1** to **4,** wherein said RNA-guided nuclease or nickase is bound to a mutated hyperactive PiggyBac transposase.

6. The composition according to any one of claims **1** to **5,** wherein the integrase of (i) and/or the integrase comprised in the POL polyprotein of (v), when present, comprises at least one amino acid mutation at a position selected from the group consisting of 10, 11, 13, 64, 94, 116, 117, 119, 120, 122, 124, 128, 152, 164, 168, 170, 185, 186, 231, 264, 266 of the HIV-1 integrase of SEQ ID NO: 1 or at a corresponding position in another integrase.

7. The composition according to any one of claims **1** to **6,** wherein the integrase of (i) and/or of (v) comprises at least one amino acid mutation selected from the group consisting of D10K, E11K, E13K, D64A, D64E, G94D, G94E, G94R, G94K, D116A, D116E, N117D, N117E, N117R, N117K, S119A, S119P, S119T, S119G, S119D, S119E, S119R, S119K, N120D, N120E, N120R, N120K, T122K, T122I, T122V, T122A, T122R, A124D, A124E, A124R, A124K, A128T, E152A, E152D, D164N, Q168L, Q168A, E170G, F185K, K186E, R231G, R231K, R231D, R231E, R231S, K264R, K266R, and K273R of the HIV-1 integrase of SEQ ID NO: 1 or at a corresponding position in another integrase.

8. The composition according to any one of claims 1 to 7, wherein the integration activity of the lentiviral vector of (i) is decreased or abolished by at least one mutation in the integrase sequence selected from the group consisting of a D116N substitution, a D164N substitution, an insertion of a premature stop codon, and a deletion of the integrase gene (ΔIN), numbering based on the HIV-1 integrase of SEQ ID NO: 1.

9. A method for producing lentiviral particles, comprising the steps of:
(i) transfecting lentiviral producer cells cultured in a suitable culture medium with the composition according to any one of claims **1** to **8,** and
(ii) collecting lentiviral particles in the culture medium of said lentiviral producer cells.

10. A population of lentiviral particles obtainable by the method according to claim **9.**

11. A population of lentiviral particles comprising lentiviral particles comprising or consisting of:
a. lentiviral proteins and/or genes or parts thereof, wherein the lentiviral integrase has impaired integration activity,
b. an RNA-guided nuclease or nickase, preferably a Cas9 protein, or a nucleic acid sequence coding therefor,
c. a guide RNA fused to an aptamer, preferably to at least one MS2 RNA tetraloop-binding sequence, and
d. optionally, a fusion protein comprising (1) a first protein comprising or consisting of a GAG polyprotein, (2) a second protein comprising or consisting of an aptamer-binding protein, and optionally (3) a third protein comprising or consisting of a POL polyprotein, or a nucleic acid sequence coding therefor.

12. An *in vitro* method for site-specific integration of a transgene of interest into the genome of a cell, comprising infecting said cell with the population of lentiviral particles according to claim **10** or **11,** wherein the population of lentiviral particles comprises a transgene of interest or wherein a transgene of interest is delivered to the cell before, concomitantly with or after the population of lentiviral particles.

13. The population of lentiviral particles according to claim **10** or **11,** for use as a drug.

14. The population of lentiviral particles according to claim **10** or **11,** for use for site-specific integration of a transgene of interest into the genome of a cell, wherein the population of lentiviral particles comprises a transgene of interest or wherein a transgene of interest is to be delivered to the cell before, concomitantly with or after the population of lentiviral particles.

15. The population of lentiviral particles according to claim **10** or **11,** for use in the treatment of a disease in a subject in need thereof, preferably of a genetic disease, wherein the population of lentiviral particles comprises a transgene of interest or wherein a transgene of interest is to be delivered to the cell before, concomitantly with or after the population of lentiviral particles.
